(19) Europäisches Patentamt
European Patent Office
Office européen des brevets

(11) **EP 3 446 617 A1**

(12) **EUROPEAN PATENT APPLICATION**
published in accordance with Art. 153(4) EPC

(43) Date of publication:
**27.02.2019 Bulletin 2019/09**

(51) Int Cl.:
***A61B 1/00*** (2006.01)  ***A61B 1/045*** (2006.01)

(21) Application number: **17785652.3**

(22) Date of filing: **28.02.2017**

(86) International application number:
**PCT/JP2017/007665**

(87) International publication number:
**WO 2017/183307 (26.10.2017 Gazette 2017/43)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **20.04.2016 JP 2016084700**

(71) Applicant: **FUJIFILM Corporation
Tokyo 106-8620 (JP)**

(72) Inventor: **ENDO Maiko
Ashigarakami-gun
Kanagawa 258-8538 (JP)**

(74) Representative: **Klunker IP
Patentanwälte PartG mbB
Destouchesstraße 68
80796 München (DE)**

(54) **ENDOSCOPE SYSTEM, IMAGE PROCESSING DEVICE, AND IMAGE PROCESSING DEVICE OPERATION METHOD**

(57) Provided are an endoscope system, an image processing device, and a method of operating the image processing device that can estimate the absolute blood vessel depth of blood vessels depicted in an endoscopic image. An endoscope system includes a light source unit that generates a plurality of types of illumination light having different wavelength ranges; an imaging sensor that images an observation target irradiated with any one of the plurality of types of illumination light; a blood vessel shape determination unit (84) that, on the basis of an image signal obtained from the imaging sensor, determines a shape pattern of blood vessels of interest that are some or all of blood vessels included in a captured image of the observation target; and a blood vessel depth estimation unit (85) that estimates a blood vessel depth of the blood vessels of interest on the basis of the shape pattern.

FIG. 21

EP 3 446 617 A1

**Description**

**BACKGROUND OF THE INVENTION**

1. Field of the Invention

The present invention relates to an endoscope system, an image processing device, and a method of operating an image processing device, and particularly, to an image processing technique and a diagnosis assisting technique that acquire information on blood vessels from an image captured by an endoscope.

2. Description of the Related Art

In examination or diagnosis using endoscope systems, the importance of information on blood vessels has been recognized. In recent years, endoscope systems that extract the information on the blood vessels by various methods are suggested (JP5393525B and JP2011-218135A). JP5393525B discloses an endoscope system that extracts blood vessels present in a mucous membrane surface layer of a living body tissue and blood vessels present in a deep layer of the living body tissue by performing weighting processing on an image obtained using blue narrowband light and an image obtained using green narrowband light, respectively.

JP2011-218135A discloses an endoscope system that radiates a plurality of types of narrowband light having mutually different wavelength ranges to a subject tissue and calculates blood vessel depth and oxygen saturation concentration on the basis of a brightness ratio between narrowband image data acquired via imaging elements for the radiation of the respective types of narrowband light.

**SUMMARY OF THE INVENTION**

As disclosed in JP5393525B and JP2011-218135A, information on the blood vessels present at the different depths can be extracted from respective images having a plurality of wavelength ranges acquired by multi-frame imaging, utilizing a plurality of types of illumination light having different wavelength ranges.

The depth of the blood vessels ascertained by the related-art methods is a relative depth showing whether the blood vessels are in the surface layer or the deep layer with reference to the mucous membrane, and is not an absolute numerical value showing an actual depth. In order to calculate the absolute depth of the blood vessels from an image obtained by extracting the blood vessels, it is necessary to know the scattering coefficient of a mucous membrane part. However, since there are individual differences in the scattering coefficient of the mucous membrane part, the absolute blood vessel depth cannot be calculated in a case where the scattering coefficient is unknown. Additionally, it is also considered that the absolute blood vessel depth is calculated by estimating the scattering coefficient of the mucous membrane part from the image acquired by the endoscope. However, since the pixel values of the acquired image of the endoscope fluctuate due to various external causes, such as radiation unevenness and quantity fluctuation of the illumination light, it is difficult to estimate the scattering coefficient of the mucous membrane part from the acquired image.

The invention has been made in view of such situations, and an object thereof is to provide an endoscope system, an image processing device, and a method of operating an image processing device that can estimate the absolute blood vessel depth of blood vessels depicted in an endoscopic image even in a case where the scattering coefficient of the observation target is unknown.

An endoscope system related to a first aspect according to one viewpoint of the present disclosure comprises a light source unit that generates a plurality of types of illumination light having different wavelength ranges; an imaging sensor that images an observation target irradiated with any one of the plurality of types of illumination light; a blood vessel shape determination unit that, on the basis of an image signal obtained from the imaging sensor, determines a shape pattern of blood vessels of interest that are some or all of blood vessels included in a captured image of the observation target; and a blood vessel depth estimation unit that estimates a blood vessel depth of the blood vessels of interest on the basis of the shape pattern.

According to the endoscope system of the first aspect, the shape pattern of the blood vessels of interest is determined from the image captured by the imaging sensor, and the blood vessel depth is estimated from the determined shape pattern. The shape of the blood vessels is various depending on the types of blood vessels, and the depth of a living body tissue in which the blood vessels are present varies with the types of blood vessels. According to the first aspect, the blood vessel depth of the blood vessels of interest can be estimated from a relationship between the shape pattern and the depth of the blood vessels.

The "image signal obtained from the imaging sensor" may be an image signal acquired in real time from the imaging sensor, or may be an image signal that is acquired via the imaging sensor and saved in a memory or other storages. Concepts of both an analog image signal and a digital image signal are included in the term "image signal".

An image signal obtained by performing a demosaicing processing, color conversion processing, and gradation transformation processing, and other various kinds of signal processing on the image signal obtained from the imaging sensor is included in the concept of "the image signal obtained from the imaging sensor". The "captured image" is an image captured by the imaging sensor. An image represented by the image signal obtained from the imaging sensor is included in the concept of the "captured image".

The "blood vessels of interest" are blood vessels used as a target from which the blood vessel depth is estimated. The blood vessels of interest may be specific blood vessels that are some blood vessels among the blood vessels within the captured image, or may be all blood vessels within the captured image.

As a second aspect, in the endoscope system of the first aspect, it is possible to adopt a configuration in which the blood vessel depth estimation unit estimates the blood vessel depth of the blood vessels of interest, utilizing correspondence information in which a blood vessel shape and a blood vessel depth are associated with each other.

For example, images and figures showing shape patterns can be used for the information on the blood vessel shape included in the correspondence information. Additionally, information on feature amounts for describing geometrical features can be used as the information on the blood vessel shape. The depth of the blood vessels included in the correspondence information can be, for example, numerical values of the depth thereof with reference to the surface of a mucous membrane.

As a third aspect, it is possible to adopt a configuration in which the endoscope system of the second aspect further comprises a database storage unit that stores a database regarding the correspondence information.

As a fourth aspect, in the endoscope system of the second aspect or the third aspect, it is possible to adopt a configuration in which the correspondence information is prepared in accordance with respective regions of the living body tissue used as the observation target, and appropriate correspondence information is referred to in accordance with a region to be observed that is the observation target.

As a fifth aspect, it is possible to adopt a configuration in which the endoscope system according to any one aspect of the first aspect to the fourth aspect further comprises a blood vessel thickness measurement unit that measures a thickness of the blood vessels from the captured image of the observation target acquired via the imaging sensor, and the blood vessel depth estimation unit estimates the blood vessel depth on the basis of information on the shape pattern of the blood vessels of interest and the thickness of the blood vessels of interest obtained by the blood vessel thickness measurement unit.

By estimating the blood vessel depth by combination of the information on the shape pattern and the thickness, higher-accuracy blood vessel depth can be estimated.

As a sixth aspect, it is possible to adopt a configuration in which the endoscope system according to any one aspect of the first aspect to the fifth aspect further comprises a blood vessel designation unit that designates the blood vessels of interest from the captured image of the observation target acquired via the imaging sensor.

As a seventh aspect, it is possible to adopt a configuration in which the endoscope system of the sixth aspect further comprises a blood vessel extraction image creation unit that creates a blood vessel extraction image obtained by extracting a blood vessel portion from the image signal obtained from the imaging sensor, and the blood vessel designation unit designates the blood vessels of interest from the blood vessel extraction image serving as the captured image.

The "extraction" is not limited to the processing of separating and taking out only the blood vessel portion, and includes concepts, such as the processing of enhancing the blood vessel portion and the processing of differentiating the blood vessel portion.

As an eighth aspect, the endoscope system of the sixth aspect or the seventh aspect further comprises a display unit that displays the image created on the basis of the image signal obtained from the imaging sensor, and the blood vessel designation unit includes an operating part for performing an operation in which a user designates the blood vessels of interest on the image displayed on the display unit.

According to the eighth aspect, the user can select the desired blood vessels within the image as the blood vessels of interest while viewing the image displayed on the display unit.

As a ninth aspect, in the endoscope system of any one aspect of the sixth aspect to eighth aspect, it is possible to adopt a configuration in which the blood vessel designation unit includes an automatic designation processing unit that automatically designates the blood vessels of interest.

According to the ninth aspect, it is possible to estimate the blood vessels of interest from observation modes, imaging conditions, and the like, and the blood vessels of interest can be automatically designated from the captured image. An aspect including a configuration in which the blood vessels of interest are automatically selected in addition to the configuration of the manual selection according to the eighth aspect is more preferable.

As a tenth aspect, in the endoscope system of the ninth aspect, it is possible to adopt a configuration in which the blood vessel designation unit automatically designates the blood vessels of interest in accordance with a wavelength range of the illumination light radiated to the observation target in a case where the observation target is imaged.

As an eleventh aspect, in the endoscope system of the tenth aspect, it is possible to adopt a configuration in which the blood vessel designation unit designates a blood vessel thinner than a regular blood vessel thickness as the blood vessels of interest in a case where the observation target is imaged using the illumination light having a wavelength range on a relatively short wavelength side among the plurality of types of illumination light.

The illumination light having the wavelength range on the short wavelength side is mainly used in a case where surface layer blood vessels are observed. Generally, since the surface layer blood vessels are thin and fine blood vessels compared to middle-depth layer blood vessels, it is possible to extract the blood vessel portion from the image captured using the illumination light having the wavelength range on the short wavelength side, and automatically designate the blood vessels of interest, using the blood vessel thickness as a determination index.

As a twelfth aspect, in the endoscope system of the tenth aspect or the eleventh aspect, it is possible to adopt a configuration in which the blood vessel designation unit designates a blood vessel thicker than a regular blood vessel thickness as the blood vessels of interest in a case where the observation target is imaged using the illumination light having a wavelength range on a relatively long wavelength side among the plurality of types of illumination light.

The illumination light having the wavelength range on the long wavelength side is mainly used in a case where middle-depth layer blood vessels are observed. Generally, since the middle-depth layer blood vessels are thick blood vessels compared to the surface layer blood vessels, it is possible to extract the blood vessel portion from the image captured using the illumination light having the wavelength range on the long wavelength side, and automatically designate the blood vessels of interest, using the blood vessel thickness as a determination index.

As a thirteenth aspect, in the endoscope system of the ninth aspect, it is possible to adopt a configuration in which the blood vessel designation unit designates a type of a blood vessel having the highest contrast among types of the blood vessels included in the captured image, as the blood vessels of interest.

The blood vessels of interest can be automatically designated using the contrast of the blood vessels within the image as a determination index.

As a fourteenth aspect, in the endoscope system of any one aspect of the first aspect to the thirteenth aspect, it is possible to adopt a configuration in which the blood vessel shape determination unit determines the shape pattern of the blood vessels of interest on the basis of information on classification patterns of a blood vessel shape determined in advance in accordance with types of the blood vessels.

As a fifteenth aspect, in the endoscope system of any one aspect of the first aspect to the fourteenth aspect, it is possible to adopt a configuration in which the blood vessel shape determination unit determines the shape pattern, using at least one feature amount of the number of branches or the number of loops of the blood vessels.

As a sixteenth aspect, it is possible to adopt a configuration in which the endoscope system of any one aspect of the first aspect to the fifteenth aspect further comprises an information presentation unit that presents information on the blood vessel depth estimated by the blood vessel depth estimation unit together with the image in which the blood vessels of interest are included.

The display unit in the eighth aspect can be made to function as the information presentation unit in the sixteenth aspect.

An image processing device related to a seventeenth aspect of according to another viewpoint of the present disclosure comprises an image signal acquisition unit that acquires an image signal that is obtained by irradiating an observation target with a plurality of types of illumination light having different wavelength ranges and by imaging the observation target, using an imaging sensor, under the irradiation of the respective types of illumination light; a blood vessel shape determination unit that determines a shape pattern of blood vessels of interest that are some or all of blood vessels included in a captured image of the observation target on the basis of the image signal acquired by the image signal acquisition unit; and a blood vessel depth estimation unit that estimates a blood vessel depth of the blood vessels of interest on the basis of the shape pattern.

In the seventeenth aspect, the same matters as the matters specified in the second aspect to the sixteenth aspect can be appropriately combined together.

A method of operating an image processing device related to an eighteenth aspect related to still another aspect of the present disclosure comprising an image signal acquisition step of acquiring an image signal that is obtained by irradiating an observation target with a plurality of types of illumination light having different wavelength ranges and by imaging the observation target, using an imaging sensor, under the irradiation of the respective types of illumination light; a blood vessel shape determination step of determining a shape pattern of blood vessels of interest that are some or all of blood vessels included in a captured image of the observation target on the basis of the image signal acquired by the image signal acquisition step; and a blood vessel depth estimation step of estimating a blood vessel depth of the blood vessels of interest on the basis of the shape pattern.

In the eighteenth aspect, the same matters as the matters specified in the second aspect to the sixteenth aspect can be appropriately combined together. In that case, elements, such as means, processing units, or operation units, which are specified in the endoscope system, can be ascertained as elements of the steps of bearing the processing, operation, or functions corresponding to these.

According to the invention, the absolute blood vessel depth can be estimated without using the information on the scattering coefficient of the observation target.

## BRIEF DESCRIPTION OF THE DRAWINGS

Fig. 1 is an external view illustrating an endoscope system according to a first embodiment.
Fig. 2 is a block diagram illustrating a schematic configuration of an endoscope system.
Fig. 3 is a graph illustrating an example of the spectroscopic spectrum of a light source.
Fig. 4 is a graph illustrating the spectral characteristics of color filters used for an imaging sensor.
Fig. 5 is a graph illustrating the scattering coefficient of an observation target.
Fig. 6 is a graph illustrating the absorption coefficient of hemoglobin.
Fig. 7 is a block diagram illustrating the functions of a special observation image processing unit.
Fig. 8 is a graph schematically illustrating a relationship between the depth of blood vessels and the contrast of the blood vessels.
Fig. 9 is an illustrative view schematically illustrating an example of assignment of signal channels in a case where a specific-depth blood vessel enhanced image is created.
Fig. 10 is a flowchart illustrating a procedure from generation of illumination light to image processing in a special observation mode.
Fig. 11 is a view illustrating an example of a captured image captured using first narrowband light having a central wavelength of 405 nm.
Fig. 12 is a view illustrating an example of a captured image captured using second narrowband light having a central wavelength of 445 nm.
Fig. 13 is a view illustrating an example of a blood vessel enhanced image created from the image illustrated in Fig. 11 and the image illustrated in Fig. 12.
Fig. 14 is a view illustrating an example of a captured image captured using the first narrowband light having a central wavelength of 405 nm.
Fig. 15 is a view illustrating an example of a captured image captured using second narrowband light having a central wavelength of 445 nm.
Fig. 16 is a view illustrating an example of a blood vessel enhanced image created from the image illustrated in Fig. 14 and the image illustrated in Fig. 15.
Fig. 17 is a view illustrating an example of a captured image captured using third narrowband light having a central wavelength of 540 nm.
Fig. 18 is a view illustrating an example of a captured image captured using fourth narrowband light having a central wavelength of 620 nm.
Fig. 19 is a view illustrating an example of a blood vessel enhanced image created from the image illustrated in Fig. 17 and the image illustrated in Fig. 18.
Fig. 20 is a view illustrating an example of a special observation image obtained by the endoscope system.
Fig. 21 is a block diagram illustrating the functions of an image processing device.
Fig. 22 is a flowchart illustrating a flow of processing of estimating the blood vessel depth in the endoscope system of the present embodiment.
Fig. 23 is a view illustrating an example of a display screen of a display unit.
Fig. 24 is a block diagram illustrating the functions of a processor device of an endoscope system according to a second embodiment.

## DESCRIPTION OF THE PREFERRED EMBODIMENTS

Hereinafter, embodiments of the invention will be described in detail according to the accompanying drawings.

[First embodiment]

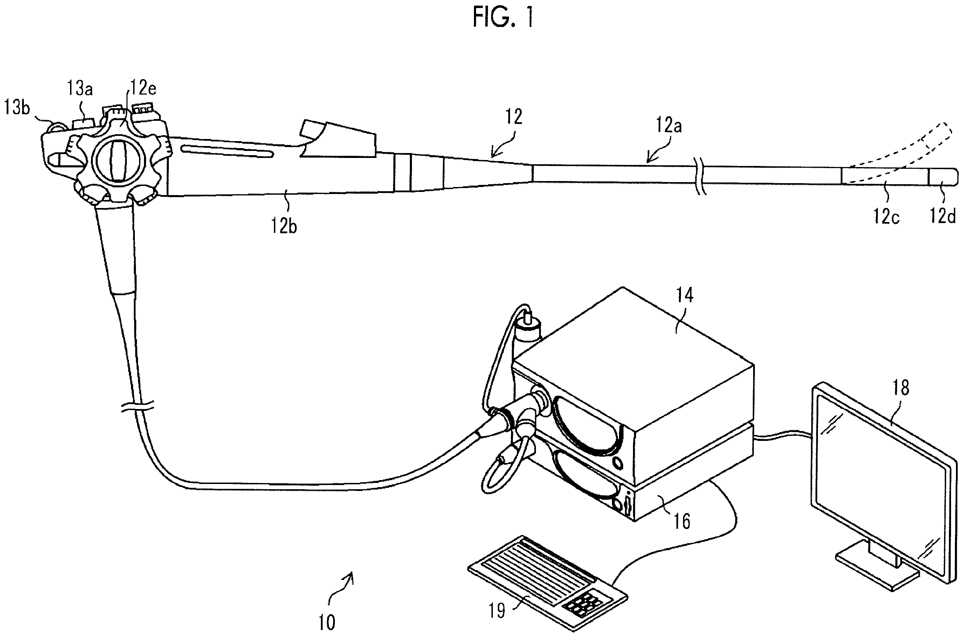

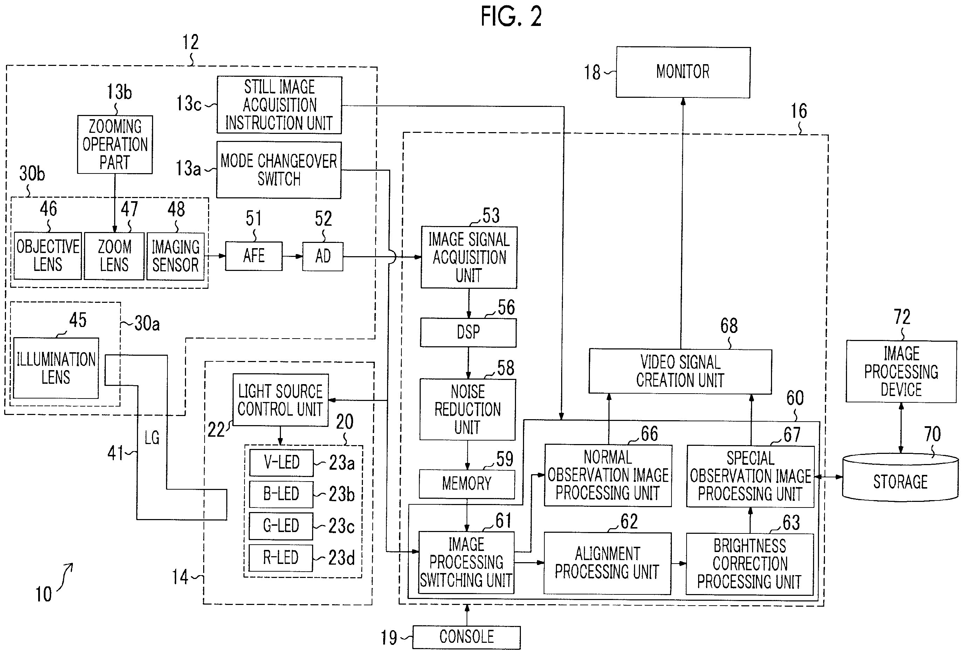

Fig. 1 is an external view illustrating an endoscope system 10 related to a first embodiment. Fig. 2 is a block diagram illustrating the functions of the endoscope system 10. As illustrated in Fig. 1, an endoscope system 10 has an endoscope 12, a light source device 14, a processor device 16, a monitor 18, and a console 19. Additionally, the endoscope system 10 of the present embodiment has a storage 70 and an image processing device 72 that are illustrated in Fig. 2. The endoscope 12 is optically connected to the light source device 14 and is electrically connected to the processor device 16.
The endoscope 12 has an insertion part 12a to be inserted into a subject, an operating part 12b provided at a proximal end portion of the insertion part 12a, and a bending part 12c and a distal end part 12d provided on a distal end side of the insertion part 12a. By operating an angle knob 12e of the operating part 12b, the bending part 12c makes a bending motion. The distal end part 12d is directed in a desired direction by this bending motion.

The angle knob 12e is provided with a mode changeover switch 13a and a zooming operation part 13b in addition to the operating part 12b. Additionally, the operating part 12b is provided with a still image acquisition instruction unit 13c that is not illustrated in Fig. 1 (refer to Fig. 2).

The mode changeover switch 13a is used for switching the operation of observation modes. The endoscope system 10 has a normal observation mode and a special observation mode as the observation modes. In the case of the normal observation mode, the endoscope system 10 displays an image obtained by imaging the observation target using white light for illumination light on the monitor 18. An image obtained by imaging the observation target in the normal observation mode is referred to as a "normal observation image". The normal observation mode can be paraphrased as a "white light observation mode". The normal observation image can be paraphrased as a "white light observation image". The illumination light can be paraphrased as "observation light".

In the case of the special observation mode, the endoscope system 10 creates a visualized image in which blood vessels in a specific depth region of the observation target are enhanced using image signals obtained by imaging the observation target, using narrowband light in a specific wavelength range for the illumination light, and displays an image suitable for observation of the blood vessels on the monitor 18. The image obtained in the special observation mode is referred to as a "special observation image". The special observation mode can be paraphrased as a "narrowband observation mode". The special observation image can be paraphrased as a "blood vessel enhanced image", a "blood vessel visualized image", or a "narrowband observation image". The endoscope 12 of the present example has a plurality of special observation modes in which the types or combinations of wavelength ranges of the narrowband light to be used are different from each other.

The processor device 16 is electrically connected to the monitor 18 and the console 19. The monitor 18 is a display device that outputs and displays the image of the observation target, information accompanying the image of the observation target, and the like. The console 19 functions as a user interface that receives input operations, such as function settings and various instructions, of the endoscope system 10. An external storage that is not illustrated in Fig. 1 may be connected to the processor device 16. The image of the observation target, the information accompanying the image, and the like can be recorded in the external storage. The storage 70 illustrated in Fig. 2 is an example of the external storage and functions as an external recording unit.

As illustrated in Fig. 2, the light source device 14 includes a light source 20, and a light source control unit 22 that controls the light source 20. The light source 20 is constituted by, for example, semiconductor light sources, such as light emitting diodes (LEDs) in a plurality of colors, a combination of a laser diode and a fluorescent body, a halogen light source, such as a xenon lamp, or appropriate combinations thereof. Additionally, optical filters for adjusting the wavelength ranges of light beams emitted from light emission sources, such as the LEDs, are included in the light source 20.

In the present embodiment, the light source 20 has four color LEDs of a violet light emitting diode (V-LED) 23a, a blue light emitting diode (B-LED) 23b, a green light emitting diode (G-LED) 23c, and a red light emitting diode (R-LED) 23d.

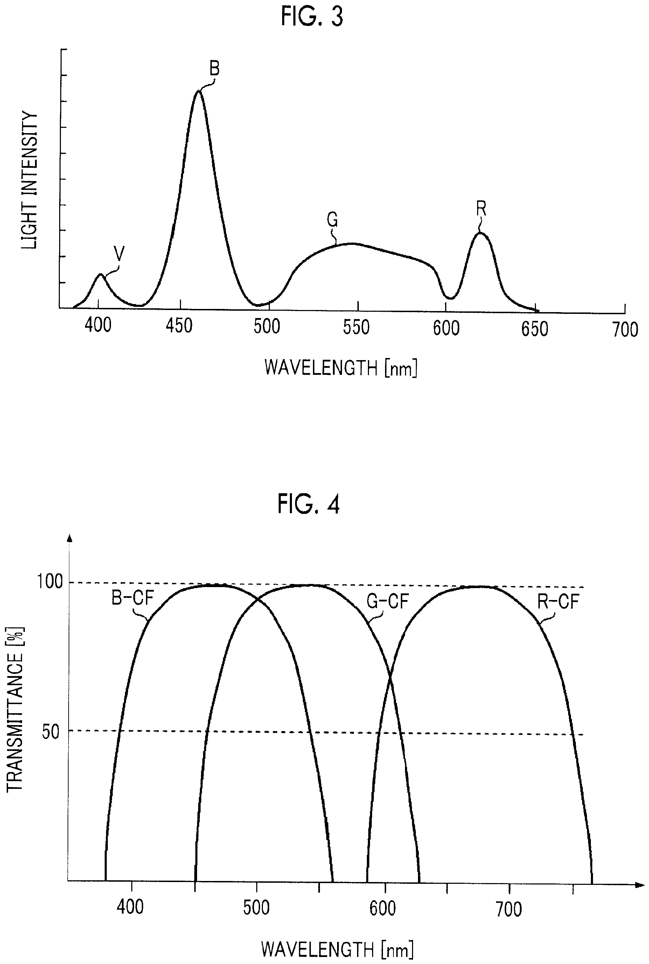

Fig. 3 is a graph illustrating an example of the spectroscopic spectrum of the light source 20. The V-LED 23a is a violet semiconductor light source that emits violet light V having a wavelength range of 380 nm to 420 nm of which the central wavelength is about 400 nm $\pm$ 10 nm. The B-LED 23b is a blue semiconductor light source that emits blue light B having a wavelength range of 420 nm to 500 nm of which the central wavelength is about 450 nm $\pm$ 10 nm. The G-LED 23c is a green semiconductor light source that emits green light G having a central wavelength of about 540 nm $\pm$ 10 nm and having a wavelength range ranging from 480 nm to 600 nm. The R-LED 23d is a red semiconductor light source that emits red light R having a central wavelength of 620 nm $\pm$ 10 nm and having a wavelength range ranging from 600 nm to 650 nm. In addition, the term "central wavelength" may be read as a peak wavelength at which the spectrum intensity is maximized.

The light source control unit 22 controls the quantity of light of the illumination light by ON and OFF of the light emission sources, such as the LEDs, and the adjustment of the driving currents and driving voltages of the LEDs. Additionally, the light source control unit 22 controls the wavelength range of the illumination light, for example, by changing the optical filters. The light source control unit 22 can independently control light emission quantities during ON and OFF of the respective LEDs 23a to 23d by individually inputting control signals to the respective LEDs 23a to 23d of the light source 20. The light source 20 creates a plurality of types of illumination light to be radiated to the observation target under the control of the light source control unit 22.

The light source 20 of the present example is capable of generating a plurality of types of narrowband light, such as violet narrowband light having a central wavelength in a violet wavelength range (a wavelength range of about 350 nm to 400 nm), blue narrowband light having a central wavelength in a blue wavelength range (wavelength range of about 400 nm to 500 nm), green narrowband light having a central wavelength in a green wavelength range (a wavelength range of about 500 nm to 600 nm), and red narrowband light having a central wavelength at a red wavelength range (a wavelength range of about 600 nm to 650 nm).

As more specific examples, the light source 20 is capable of generating narrowband light, such as violet narrowband light having a central wavelength of 405 nm, blue narrowband light having a central wavelength of 445 nm, green narrowband light having a central wavelength of 540 nm, and red narrowband light having a central wavelength of 620 nm. Additionally, the light source 20 is capable of generating blue narrowband light having a central wavelength of 470 nm and is also capable of generating two or more types of blue narrowband light having different central wavelengths. Two or more types of narrowband light having different central wavelengths can also be generated regarding the violet narrowband light, the green narrowband light, and the red narrowband light, respectively. The central wavelengths of the respective types of narrowband light can be designated, for example, by changing the optical filters.

In the present disclosure, there is a case where the violet narrowband light having a central wavelength of 405 nm generated by the light source 20 may be written as the "violet light V". Additionally, there are cases where the blue narrowband light having a central wavelength of 445 nm is written as the "blue light B", the green narrowband light having a central wavelength of 540 nm is written as the "green light G", and the red narrowband light having a central wavelength of 620 nm is written as the "red light R.

In a case where the special observation mode is selected, the light source 20 generates at least two or more types of narrowband light having mutually different central wavelengths among a plurality of types of narrowband light, and the observation target to which each narrowband light is irradiated is imaged the imaging sensor 48. Hence, in the special observation mode, a plurality of kinds of endoscopic images corresponding to the types of narrowband light are obtained. In the present embodiment, in the case of the special observation mode, the light source 20 can alternately generate two types of narrowband light including first narrowband light and second narrowband light having mutually different central wavelengths. The first narrowband light of these two types of narrowband light is narrowband light on a relatively short wavelength side, and the second narrowband light is narrowband light on a relatively long wavelength side. That is, the central wavelength of the second narrowband light has a wavelength range longer than the central wavelength of the first narrowband light. For example, the first narrowband light is violet the narrowband light having a central wavelength of 405 nm, and the second narrowband light is the blue narrowband light having a central wavelength of about 445 nm.

Additionally, in the case of another special observation mode in which a combination of the third narrowband light and the fourth narrowband light different from the combination of the first narrowband light and the second narrowband light is used, the light source 20 can alternately generate two types of narrowband light including the third narrowband light and the fourth narrowband light having mutually different central wavelengths. The third narrowband light of these two types of narrowband light is narrowband light on a relatively short wavelength side and the fourth narrowband light is narrowband light on a relatively long wavelength side. That is, the central wavelength of the third narrowband light has a wavelength range longer than the central wavelength of the fourth narrowband light. For example, the third narrowband light is the green narrowband light having a central wavelength of 540 nm, and the fourth narrowband light is the red narrowband light having a central wavelength of about 620 nm.

In the present embodiment, four types of narrowband light including the first narrowband light, the second narrowband light, the third narrowband light, and the fourth narrowband light are exemplified in the order of shorter central wavelength, and a form in which these narrowband lights are selectively switched to create the special observation image will be described. In addition, in a case where the invention is carried out, the type of narrowband light is not limited to this example, and it is also possible to adopt a form in which many types of narrowband light are used.

Additionally, the light source 20 can generate the white light. In the case of the normal observation mode, the light source control unit 22 turns on the V-LED 23a, the B-LED 23b, the G-LED 23c, and the R-LED 23d altogether. For this reason, in the normal observation mode, the white light having a wide wavelength range including the violet light V, the blue light B, the green light G, and the red light R is used as the illumination light. The light source device 14 is equivalent to one form of a "light source unit".

The illumination light emitted from the light source 20 enters a light guide 41 via a light path coupling part formed of a mirror, a lens, or the like that is not illustrated. The light guide 41 is built in the endoscope 12 and a universal cord. The universal cord is a cord that connects the endoscope 12, and the light source device 14 and the processor device 16 to each other. The light guide 41 is inserted into the insertion part 12a and propagates the illumination light generated by the light source 20 up to the distal end part 12d of the endoscope 12.

The distal end part 12d of the endoscope 12 is provided with an illumination optical system 30a and an imaging optical system 30b. The illumination optical system 30a has an illumination lens 45. The illumination light propagated by the light guide 41 is radiated to the observation target via the illumination lens 45. The imaging optical system 30b has an objective lens 46, a zoom lens 47, and an imaging sensor 48. Various types of light, such as reflected light, scattered light, and fluorescence from the observation target resulting from radiating the illumination light, enter the imaging sensor 48 via the objective lens 46 and the zoom lens 47. Accordingly, the image of the observation target is focused on the imaging sensor 48. The zoom lens 47 is freely moved between a telephoto end and a wide end in accordance with the operation of the zooming operation part 13b, and enlarges or reduces the image of the observation target to be focused on the imaging sensor 48.

The imaging sensor 48 is a color imaging sensor in which any of color filters in R (red), G (green), and B (blue) is provided for each pixel. The imaging sensor 48 images the observation target to output image signal of respective RGB color channels. As the imaging sensor 48, a charge coupled device (CCD) imaging sensor or a complementary metal-oxide semiconductor (CMOS) imaging sensor is available. Additionally, instead of the imaging sensor 48 provided with color filters in original colors, a complementary color imaging sensor including complementary color filters in C (cyan), M (magenta), Y (yellow), and G (green) may be used. In a case where the complementary color imaging sensor is used, image signals of four colors of CMYG are output. For this reason, the same RGB image signals as those in the imaging sensor 48 can be obtained by converting the image signals of four colors of CMYG into image signals of three colors of RGB through color conversion between complementary colors and original colors. Additionally, instead of the imaging sensor 48, a monochrome sensor that is not provided with the color filters may be used.

Fig. 4 is a graph illustrating the spectral characteristics of the color filters used for the imaging sensors 48. A lateral axis represents wavelength and a vertical axis represents transmittance. In Fig. 4, B-CF shows characteristics of the B color filter, G-CF shows spectral characteristics of the G color filter, and R-CF shows spectral characteristic of the R color filter. The light of a wavelength range of blue from violet is received by a B pixel provided with the B color filter in the imaging sensor 48. The light of a wavelength range of green is received by a G pixel provided with the G color filter in the imaging sensor 48. The light of a wavelength range of red is received by a R pixel provided with the R color filter in the imaging sensor 48. Signals according to the quantities of light received are output from the pixels of the respective colors of RGB of the imaging sensor 48.

For example, in the special observation mode, in a case where the first narrowband light in the violet wavelength range is used as the illumination light, the imaging sensor 48 images the observation target to which the first narrowband light is radiated, and outputs a first image signal corresponding to the first narrowband light from the B pixel. Additionally, in the special observation mode, in a case where the second narrowband light in the blue wavelength range is used as the illumination light, the imaging sensor 48 outputs a second image signal corresponding to the second narrowband light from the B pixel.

The endoscope 12 includes an analog front end (AFE) circuit 51 and an analog to digital (AD) converter 52. The image signals output from the imaging sensor 48 is input to the AFE circuit 51. The AFE circuit 51 includes a correlated double sampling (CDS) circuit and an automatic gain control (AGC) circuit. The AFE circuit 51 performs the correlated double sampling and the automatic gain control on the analog image signals obtained from the imaging sensor 48. The image signals that have passed through the AFE circuit 51 are converted into digital image signals by the AD converter 52. The digital image signals after the analog-to-digital (AD) conversion are input to the processor device 16. In addition, a form in which the AD converter 52 is mounted on the AFE circuit 51 is also possible.

The processor device 16 includes the image signal acquisition unit 53, a digital signal processor (DSP) 56, a noise reduction unit 58, a memory 59, a signal processing unit 60, and a video signal creation unit 68.

The image signal acquisition unit 53 acquires the digital image signals from the endoscope 12. The DSP 56 performs various kinds of signal processing, such as defect correction processing, offset processing, gain correction processing, linear matrix processing, gamma conversion processing, demosaicing processing, and the like, on the acquired image signals via the image signal acquisition unit 53. In the defect correction processing, a signal of a defective pixel of the imaging sensor 48 is corrected. In the offset processing, dark current components are removed from the image signals subjected to the defect correction processing, and accurate zero levels are set. In the gain correction processing, a signal level is adjusted by multiplying the image signals after the offset processing by a specific gain.

The linear matrix processing for enhancing color reproducibility is performed on the image signals after the gain correction processing. Thereafter, brightness and saturation are adjusted by the gamma conversion processing. The demosaicing processing is performed on the image signals after the gamma conversion processing, and a signal of a color that runs short in each pixel is created by interpolation. The demosaicing processing is also referred to as equalization processing or synchronization processing. By means of the demosaicing processing, all pixels have respective RGB color signals.

The noise reduction unit 58 performs noise reduction processing on the image signals subjected to the demosaicing processing or the like by the DSP 56, and reduces noise. As the noise reduction processing, for example, processing performed by a moving average method, a median filter method, or the like can be adopted. The image signals from which noise is reduced by the noise reduction unit 58 are stored in the memory 59.

The signal processing unit 60 acquires the image signals after the noise reduction from the memory 59. The signal processing unit 60 performs signal processing, such as color conversion processing, color enhancement processing, and structure enhancement processing, on the acquired image signals, if necessary, and creates an endoscopic image in color on which the observation target appears. The color conversion processing is the processing of performing conversion of colors on the image signals through 3x3 matrix processing, gradation transformation processing, three-dimensional look-up table (LUT) processing, and the like. The color enhancement processing is performed on the image signals subjected to the color conversion processing. The structure enhancement processing is, for example, the processing of enhancing specific tissue and structure included in the observation target, such as blood vessels and pit patterns, and is performed on the image signals after the color enhancement processing.

The contents of the processing in the signal processing unit 60 vary depending on the observation modes. In a case where an observation mode is the normal observation mode, the signal processing unit 60 performs the signal processing in which the observation target has a natural tone, and creates the normal observation image. In a case where an observation mode is the special observation mode, the signal processing unit 60 performs the signal processing of enhancing at least the blood vessels of the observation target and creates the special observation image.

The signal processing unit 60 includes an image processing switching unit 61, a normal observation image processing unit 66, a special observation image processing unit 67, an alignment processing unit 62, and a brightness correction processing unit 63, and performs signal processing corresponding to the respective modes of the normal observation mode and the special observation mode.

The image processing switching unit 61 switches execution of creation processing of the normal observation image or creation processing of the special observation image in accordance with settings of the observation modes by the mode changeover switch 13a. In a case where the normal observation mode is set by the operation of the mode changeover switch 13a, the image processing switching unit 61 transmits the image signals received from the memory 59, to the normal observation image processing unit 66. In a case where the special observation mode is set by the operation of the mode changeover switch 13a, the image processing switching unit 61 transmits the image signals received from the memory 59, to the special observation image processing unit 67 via the alignment processing unit 62 and the brightness correction processing unit 63.

The normal observation image processing unit 66 operates in a case where the normal observation mode is set. The normal observation image processing unit 66 performs the color conversion processing, the color enhancement processing, and the structure enhancement processing on the image signals captured by irradiating the observation target with the white light, and creates normal observation image signals. A color image obtained using the normal observation image signals is the normal observation image.

The special observation image processing unit 67 is an image processing unit that operates in a case where the special observation mode is set. The special observation image processing unit 67 extracts blood vessels at a specific depth, using the first image signal obtained by irradiating the observation target with one narrowband light on the relatively short wavelength side out of two types of narrowband light having different wavelength ranges and using the second image signal obtained by irradiating the observation target with the other narrowband light on the relatively long wavelength side, and creates the special observation image representing the extracted blood vessels depending on color differences with respect to the other blood vessels.

Although a case where the first narrowband light that is the violet narrowband light having a central wavelength of 405 nm and the second narrowband light that is the blue narrowband light having a central wavelength of 445 nm are used as the two types of narrowband light having different wavelength ranges is described herein as an example, the same applies to not only the combination of the first narrowband light, the second narrowband light but also the combination of the third narrowband light that is the green narrowband light of a central wavelength of 540 nm and the fourth narrowband light that is the red narrowband light having a central wavelength of 620 nm.

The first image signal and the second image signal are input to the special observation image processing unit 67 via the alignment processing unit 62 and the brightness correction processing unit 63. The alignment processing unit 62 performs alignment between the observation target represented by the first image signal and the observation target represented by the second image signal, which are sequentially acquired. The relative positions between the images of the first image signal and the second image signal are correlated with each other by the alignment processing of the alignment processing unit 62, and the same image range can be taken out from each of the first image signal and the second image signal. The alignment processing unit 62 may correct image positions regarding only any one of the first image signal or the second image signal, or may correct the image positions regarding both the image signals. In the present example, the processing of aligning the second image signal with the first image signal with reference to the first image signal is performed.

The brightness correction processing unit 63 corrects the brightness of at least one of the first image signal or the second image signal such that the brightness of the first image signal and the brightness of the second image signal aligned by the alignment processing unit 62 have a specific ratio. For example, since the radiated light quantity ratio of the two types of narrowband light used for the special observation mode is known, the gain correction of causing the brightness of the first image signal and the brightness of the second image signal to coincide with each other is performed in order to obtain the brightness of images in cases where the observation target is irradiated with the first narrowband light and the second narrowband light having the equal quantity of light, respectively, using this irradiated light quantity ratio. Additionally, for example, the brightness correction processing unit 63 calculates the brightness of the image of the observation target represented by the first image signal by calculating an average value of pixel values of all pixels having the first image signal or of an average value of pixel values of a specific pixel region, and calculating the brightness of the image of the observation target represented by the second image signal by calculating an average value of pixel values of all pixels having the second image signal or an average value of pixel values of a specific pixel region. Then, a gain for causing the brightness of the image of the observation target represented by the first image signal and the brightness of the image of the observation target represented by the second image signal to coincide with each other is calculated, and at least one of a pixel value of the first image signal or a pixel value of the second image signal is corrected using the calculated gain.

The special observation image processing unit 67 performs the signal processing of enhancing the blood vessels of the observation target from the first image signal and the second image signal on which the brightness correction is performed, and creates the special observation image. For example, in the special observation image created by the special observation image processing unit 67, blood vessels (so-called surface layer blood vessels) at a relatively shallow position within the observation target with reference to the surface of a mucous membrane have, for example, a magenta-based color, such as a brown color, and blood vessels at a relatively deep position within the observation target with reference to the surface of the mucous membrane have, for example, a cyan-based color, such as a green color. For this reason, the blood vessels of the observation target are enhanced with differences in color with respect to the mucous membrane represented by a pink-based color. In addition, the blood vessels present at the relatively shallow position with reference to the surface of the mucous membrane are referred to as "surface layer blood vessels". In addition, particularly blood vessels present at an extremely shallow position close to the surface of the mucous membrane among the surface layer blood vessels are referred to as "extreme surface layer blood vessels". Additionally, the blood vessels present at the relatively deep position with reference to the surface of the mucous membrane are referred to as "middle-depth layer blood vessels".

The signal processing unit 60 inputs the created endoscopic image to the video signal creation unit 68. The video signal creation unit 68 converts the endoscopic image into video signals for being output to and displayed on the monitor 18. The endoscopic image created by the signal processing unit 60 can be displayed on the monitor 18 via the video signal creation unit 68.

In a case where the still image acquisition instruction unit 13c is operated to input a release instruction, the signal processing unit 60 performs the processing of saving the created endoscopic image in the storage 70. Additionally, the signal processing unit 60 can save any of the image signals read from the memory 59, the image signals processed by the alignment processing unit 62, or the image signals processed by the brightness correction processing unit 63, or a combination thereof in the storage 70.

The storage 70 is the external storage connected to the processor device 16. The storage 70 may be connected to the processor device 16 via a communication line, such as a local area network (LAN). The storage 70 is, for example, a file server of a system, such as a picture archiving and communication system (PACS), which files the endoscopic image, a network attached storage (NAS), or the like. The endoscopic image saved in the storage 70 can be used by the image processing device 72.

The image processing device 72 is a device that has a function of performing image processing on the endoscopic image to estimate blood vessel depth. The image processing device 72 functions as a diagnosis assisting device that performs image processing on the endoscopic image to calculate blood vessel parameters for diagnosis assistance.

[Method of creating special observation image]

First, a method of creating the special observation image in the endoscope system 10 will be described. In the special observation mode, depths under the mucous membrane where observable blood vessels are present are approximately determined depending on the depth of reach of the illumination light to be radiated in a case where the observation target is imaged. Generally, light having short wavelength has a smaller depth of reach, scattered and absorbed in the vicinity of the surface of the mucous membrane, and a portion of the light is observed as reflected light. The light absorption and scattering characteristics of living body tissue that is the observation target have wavelength dependability, and the depth of reach is larger as light has longer wavelength.

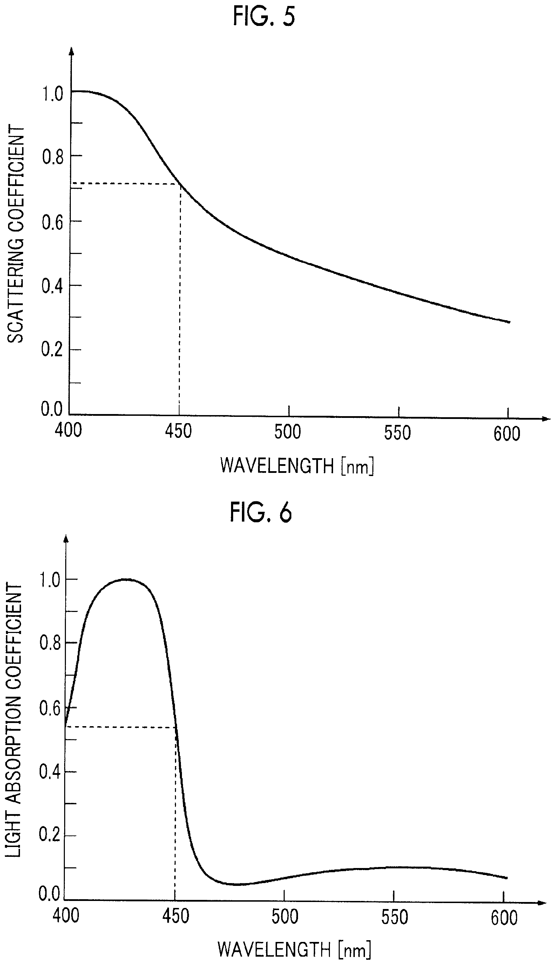

Fig. 5 is a graph illustrating the scattering coefficient of the observation target. A lateral axis of Fig. 5 represents wavelength and a vertical axis represents standardized scattering coefficient. As illustrated in Fig. 5, as the wavelength is shorter, the scattering coefficient is larger. As the scattering is larger, the light reflected in the vicinity of a mucous membrane surface layer of the living body tissue is larger, and the light that reaches the middle-depth layer is smaller. For that reason, as the wavelength is shorter, the depth of reach is smaller, and as the wavelength is longer, the depth of reach is larger. Although the scattering coefficient has individual differences, the tendency of the wavelength dependability is common.

The scattering coefficients of the observation target in respective wavelength ranges of a plurality of types of narrowband light used in the special observation mode relate to the depths of reach of the respective types of narrowband light, that is, depths under the mucous membrane, of blood vessels observable in the wavelength ranges. However, as already described, it is difficult to ascertain accurate scattering coefficients from the endoscopic image regarding an actual observation target.

Meanwhile, the absorption coefficients of hemoglobin in the wavelength ranges of the respective types of narrowband light relate to the contrast of blood vessels observable with the respective types of narrowband light.

Fig. 6 is a graph illustrating the absorption coefficient of hemoglobin. A lateral axis of Fig. 6 represents wavelength and a vertical axis represents standardized absorption coefficient. As understood from Fig. 6, the short-wavelength light has large hemoglobin absorption and also large light scattering (refer to Fig. 5). For this reason, although an image captured by radiating the short-wavelength narrowband light has a high contrast of blood vessels at a shallow position, the contrast of blood vessels at a deep position becomes sharply low. Meanwhile, as the narrowband light to be used for the illumination light has longer wavelength, the contrast of the blood vessels at the shallow position becomes low. However, a decrease in the contrast of the blood vessels at the deep position becomes relatively gentle. Blood vessel information at any depth can be visualized from difference information on two images captured by changing the wavelength of the illumination light by utilizing such characteristics.

For example, by allocating one of the images obtained by performing imaging by radiating illumination light of two types wavelength ranges including the violet narrowband light having a central wavelength of 405 nm and the blue narrowband light having a central wavelength of 445 nm, respectively, to brightness signals as the illumination light on the short wavelength side to allocate a difference image between both the images to color difference signals, blood vessels present in the surface layer of the mucous membrane can be extracted, and an image in which the extracted blood vessels are enhanced can be drawn up.

Additionally, for example, in a case where illumination light of two types of wavelength ranges including the green narrowband light having a central wavelength of 540 nm and the red narrowband light having a central wavelength of 620 nm as the illumination light by long wavelength side, blood vessels in a deeper layer under the mucous membrane can be extracted, and an image in which the extracted blood vessels are enhanced is drawn up.

It is desirable that the two types of illumination light to be used in the special observation mode are light of wavelength ranges in which the scattering coefficients of the observation target are different from each other and the light absorption coefficients of hemoglobin are substantially equal to each other. By using the two types of illumination light that satisfy such conditions, the blood vessels at the specific depth under the mucous membrane can be particularly clearly extracted.

Hence, the conditions that "the scattering coefficients of the observation target are different from each other and the light absorption coefficients of hemoglobin are substantially equal to each other" mean conditions that light of two wavelength ranges in which the depths (depths of reach) under the mucous membrane, of the observable blood vessels are different from each other and blood vessels having different depths under the mucous membrane are observable with the same degree of contrast is selected and used.

In addition, in the first narrowband light having a central wavelength of 405 nm and the second narrowband light having a central wavelength of 445 nm that are used in the present embodiment, as illustrated in Fig. 6, the light absorption coefficients (Light absorption coefficient of oxygenated hemoglobin : Light absorption coefficient of reduced hemoglobin = 3:7) of hemoglobin are approximately equal to each other. The combination of the first narrowband light having a central wavelength of 405 nm and the second narrowband light having a central wavelength of 445 nm is an example of a preferable combination for the extraction of blood vessels.

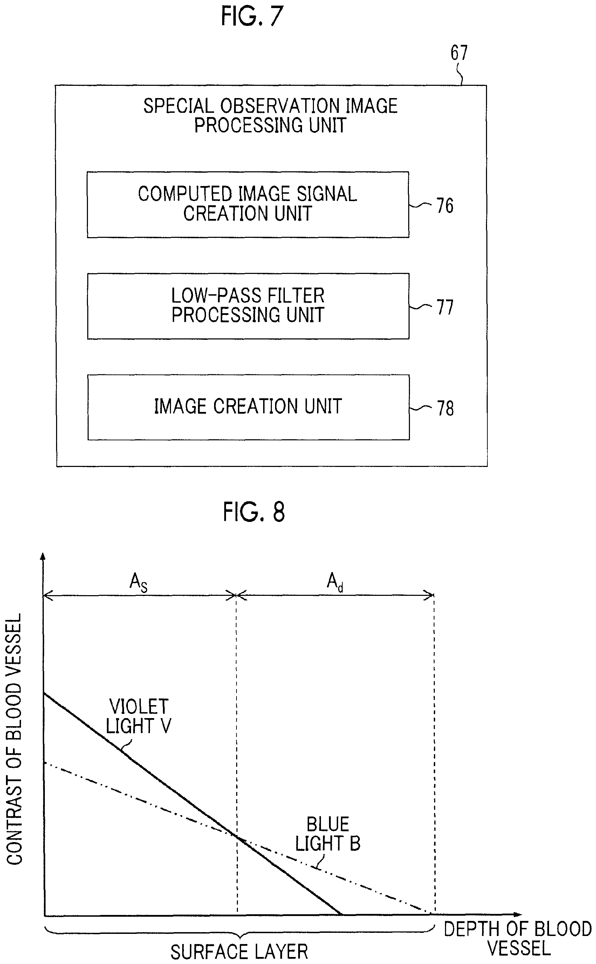

Fig. 7 is a block diagram illustrating the functions of the special observation image processing unit 67. The special observation image processing unit 67 includes a computed image signal creation unit 76, a low-pass filter (LPF) processing unit 77, and an image creation unit 78.

The computed image signal creation unit 76 performs computation using the first image signal and the second image signal subjected to the alignment processing and the brightness correction processing, and creates commutated image signals. Specifically, a difference or ratio between the first image signal and the second image signal is calculated. The computed image signal creation unit 76 of the present example log-transforms the first image signal and the second image signal, respectively, and creates a difference between the first image signal and the second image signal after the logarithmic transformation, more specifically, and a computed image signal ΔB obtained by subtracting the first image signal from the second image signal. The logarithmic transformation is also referred to as "Log transformation".

The first image signal and the second image signal have pixel values proportional to densities in a case where these signals are log-transformed, although respective pixels have pixel values proportional to received light quantities. Thus, stable computation results can be obtained irrespective of the illuminance of illumination light in a case where respective image signals are obtained. In the present embodiment, it is supposed that there is no substantial difference in the illuminance of the first narrowband light and the second narrowband light to be used as the illumination light in the special observation mode, and a computed image signal ΔB is created due to the difference between the first image signal and the second image signal as described above.

In addition, in a case where the first image signal and the second image signal are used as they are without log-transforming the respective image signals of the first image signal and the second image signal, the computed image signal may be created by calculating the ratio of the first image signal and the second image signal for each pixel.

Fig. 8 is a graph schematically illustrating a relationship between the depth of blood vessels and the contrast of the blood vessels. As illustrated in Fig. 8, in a case where two types of light the violet light V and the blue light B are used as the illumination light, it is possible to observe blood vessels within a total range of depth $A_s$ and depth $A_d$, that is, blood vessels that are approximately present in the surface layer (surface layer blood vessels). However, since the violet light V has a wavelength shorter than the blue light B, the depth of reach to the observation target is low, and only blood vessels present in the depth range $A_s$ at the relatively shallow position under the mucous membrane with respect to the blue light B are projected, whereas the contrast of the blood vessels present in the depth range $A_s$ at the shallow position is larger than that in a case where the blue light B is used. The "contrast of blood vessels" means the ratio of the quantity of reflected light from a surrounding mucous membrane to the quantity of reflected light from the blood vessels. The contrast of blood vessels can be calculated by, for example, "$Y_V/Y_M$" or "$(Y_V - Y_M)/(Y_V + Y_M)$", using the brightness $Y_V$ of blood vessels, and the brightness $Y_M$ of the mucous membrane.

Meanwhile, since the blue light B has a wavelength longer than the violet light V, the depth of reach to the observation target is high, and only blood vessels present in the depth range $A_d$ at the relatively deep position under the mucous membrane with respect to the violet light V are projected, whereas the contrast of the blood vessels present in the depth range $A_s$ at the shallow position is smaller than that in a case where the violet light V is used.

For this reason, in a case where the first image signal corresponding to the violet light V is subtracted from the second image signal corresponding to the blue light B, the pixel values of pixels representing particularly the extreme surface layer blood vessels present in the depth range $A_s$ at the shallow position under the mucous membrane are enhanced and become large values (white). On the contrary, the pixel values of pixels representing the blood vessels present at the depth range $A_d$ at a position deeper than the extreme surface layer blood vessels become small values (black). Calculating the computed image signal $\Delta B$ corresponds to extracting the blood vessels at the specific depth under the mucous membrane.

The low-pass filter processing unit 77 performs resolution reducing processing by applying a low-pass filter to the computed image signal $\Delta B$ created by the computed image signal creation unit 76. The intensity of the filter processing that the low-pass filter processing unit 77 performs on the computed image signal $\Delta B$ is determined by the cut-off frequency of the low-pass filter. The cut-off frequency of the low-pass filter is set in advance, and the sharpness thereof is made lower than the sharpness of at least an original computed image signal $\Delta B$. The computed image signal obtained by the low-pass filter processing of the low-pass filter processing unit 77 becomes an image in a further blurred state than the original computed image signal.

The image creation unit 78 creates an image having a plurality of output channels, using either the first image signal or the second image signal received by the special observation image processing unit 67 and the computed image signal $\Delta B$ subjected to that low-pass filter processing. More specifically, the image creation unit 78 creates an image having a brightness channel Y and two color difference channels Cb and Cr related to color differences. The brightness channel Y is equivalent to the first channel, and the two color difference channels Cb and Cr are equivalent to the second channel and the third channel, respectively. The image creation unit 78 allocates either the first image signal or the second image signal to the brightness channel Y and allocates the resolution-reduced computed image signal $\Delta B$ subjected to the low-pass filter processing to the two color difference channels Cb and Cr, thereby creating an image in which the pattern of the blood vessels at the specific depth is enhanced in colors. A YCC image created in this way or an RGB image obtained by performing color conversion processing of the YCC image is referred to as a "blood vessel enhanced image". The blood vessel enhanced image is also referred to as the "blood vessel visualized image". The "YCC image" means a color image represented by a Y signal that is a brightness signal, and a Cr signal and a Cb signal that are color difference signals.

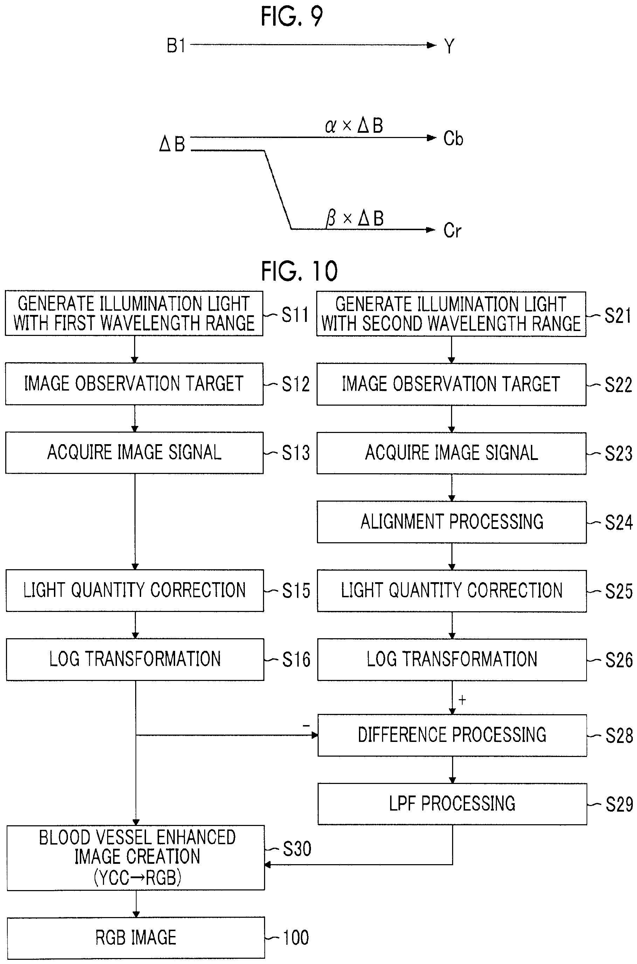

Fig. 9 is an illustrative view schematically illustrating an example of assignment of the signal channels in a case where the specific-depth blood vessel enhanced image is created. B1 in Fig. 9 represents the first image signal. In the case of the present embodiment, the first image signal corresponding to the narrowband light (violet light V) in the relatively short wavelength range out of the first image signal and the second image signal is allocated to the brightness channel Y. That is, the first image signal having a relatively high contrast of the extreme surface layer blood vessel is allocated to the brightness channel Y. Also, the computed image signal $\Delta B$ is allocated to the color difference channels Cb and Cr. In a case where the computed image signal $\Delta B$ is allocated to the color difference channels Cb and Cr, multiplication is made by a coefficient $\alpha$ and a coefficient $\beta$, respectively. This is for aligning an image and tone to be displayed by an endoscope system that enhances and observes the surface layer blood vessels or the like. The first image signal is allocated to the brightness channel Y in order to classify and enhance the extreme surface layer blood vessels out of the surface layer blood vessel.

In the endoscope system having the observation mode in which the surface layer blood vessels are enhanced and observed, as one of the methods of creating a surface layer blood vessel enhanced image, there is the following method using a B image signal and a G image signal of a captured image. That is, in the case of a surface layer blood vessel observation mode, narrow-band blue light is radiated to image the observation target to acquire the B image signal, and narrow-band green light is radiated to image the observation target to acquire the G image signal. Then, by allocating the B image signal to a B channel and a G channel of a display image and allocating the G image signal to an R channel, the middle-depth layer blood vessels at the deep position under the mucous membrane are turned into a green-based (cyan-based) color, and the surface layer blood vessels at the shallow position under the mucous membrane are turned into a red-based (magenta-based) color and are enhanced and displayed.

In ITU-R.601 that is the standard of the International Telecommunications Union, a relationship between the respective RGB image signals, the brightness channel Y, and the color difference channels Cb and Cr is expressed by the following Equations (1), (2), and (3). In addition, the ITU is an abbreviated notation of "International Telecommunication Union".

$$Y = 0.299R + 0.587G + 0.114B \qquad (1)$$

$$Cb = -0.169R - 0.331G + 0.5B \qquad (2)$$

$$Cr = 0.5R - 0.419G - 0.081B \qquad (3)$$

Then, in Equation (2) and Equation (3) of the color difference channels Cb and Cr, in a case where G is substituted for R and B is substituted for G, the color difference channels Cb and Cr can be expressed with (G - B) as shown in Equation (4) and Equation (5).

$$Cb = -0.169G + 0.169B = -0.169 \, (G - B) \qquad (4)$$

$$Cr = 0.5G - 0.5B = 0.5 \, (G - B) \qquad (5)$$

With respect to the above-described method, in the special observation mode in the present embodiment, in order to obtain the first image signal and the second image signal, using the first narrowband light in the violet wavelength range and the second narrowband light in the blue wavelength range to extract and display the extreme surface layer blood vessels, the computed image signal ΔB is used instead of the signal (G - B) of Equation (4) and Equation (5). That is, multiplication is made by the coefficient α = -0.169 to allocate the computed image signal ΔB to the color difference signal Cb, and multiplication is made by the coefficient β = 0.5 to allocate the computed image signal ΔB to the color difference signal Cr.

Accordingly, in the special observation mode of the endoscope system 10 of the present embodiment, the blood vessel enhanced image of almost the same color scheme as the surface layer blood vessel enhanced image obtained by above-described surface layer blood vessel observation mode can be obtained. Here, in the present embodiment, in order to enhance color differences between the extreme surface layer blood vessels and the surface layer blood vessels at the relatively deep position, there is a case where the coefficient α and the coefficient β may be further multiplied by coefficients in accordance with settings or the like.

In addition, in order to create the blood vessel enhanced image of RGB from the brightness channel Y and the color difference channels Cb and Cr, the following Equations (6), (7), and (8) are satisfied in accordance with the inverse transformation of ITU-R.601.

$$R = Y + 1.402Cr \qquad (6)$$

$$G = Y - 0.344Cb - 0.714Cr \qquad (7)$$

$$B = Y + 1.772Cb \qquad (8)$$

The specific-depth blood vessel enhanced image created by the special observation image processing unit 67 in this way is input to the video signal creation unit 68. The video signal creation unit 68 converts the specific-depth blood vessel enhanced image into video signals for display as an image that can be displayed by the monitor 18. The specific-depth blood vessel enhanced image is displayed on the monitor 18, using the video signals.

[Outlines of image processing in special observation mode]

Fig. 10 is a flowchart illustrating a procedure from generation of the illumination light to image processing in the special observation mode. In a case where the special observation mode is selected, the image processing illustrated in Fig. 10 is executed by the processor device 16. In Step S11, the light source 20 generates the illumination light that is the narrowband light having the first wavelength range. The first wavelength range is, for example, the violet wavelength range having a central wavelength of 405 nm. The illumination light emitted from the light source 20 in Step S11 is referred to as first illumination light. The first illumination light emitted from the light source 20 is radiated to the observation target.

In Step S12, the imaging sensor 48 images the observation target to which the first illumination light is radiated, and outputs an image signal corresponding to the first illumination light.

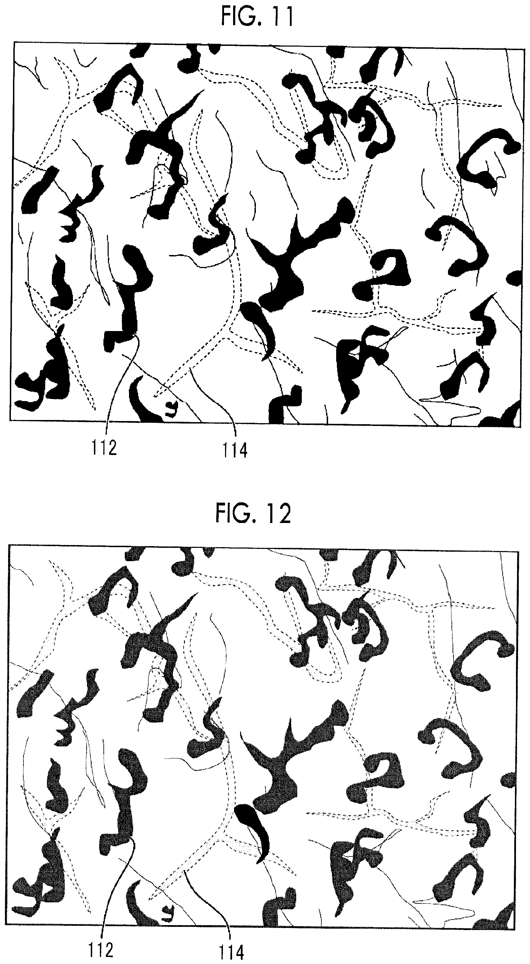

In Step S13, the image signal acquisition unit 53 acquires the image signal corresponding to the first illumination light from the imaging sensor 48. The image signal acquired in Step S13 is equivalent to the first image signal that is already described. An example of a captured image obtained in Step S13 is illustrated in Fig. 11. Fig. 11 shows the example of the captured image captured using the first narrowband light having a central wavelength of 405 nm. In Fig. 11, surface layer blood vessels including extreme surface layer blood vessels 112 are clearly projected.

In Step S15 of Fig. 10, the brightness correction processing unit 63 performs light quantity correction on the acquired first image signal. The light quantity correction is the processing of correcting the brightness of the entire image according to the quantity of the illumination light. The light quantity correction is synonymous with the "brightness correction processing".

In Step S16, the computed image signal creation unit 76 performs log transformation on the first image signal subjected to the light quantity correction.

Additionally, the light source 20 generates the illumination light that is the narrowband light having the second wavelength range after Step S13 (Step S21). The second wavelength range is the blue wavelength range having a central wavelength of 445 nm. The illumination light emitted from the light source 20 in Step S21 is referred to as second illumination light. The second illumination light emitted from the light source 20 is radiated to the observation target.

In Step S22, the imaging sensor 48 images the observation target to which the second illumination light is radiated, and outputs an image signal corresponding to the second illumination light.

In Step S23, the image signal acquisition unit 53 acquires the image signal corresponding to the second illumination light from the imaging sensor 48. The image signal acquired in Step S23 is equivalent to the second image signal that is already described.

In Step S24, the alignment processing unit 62 performs alignment processing of the first image signal acquired in Step S13 and the second image signal acquired in Step S23. In the present example, the processing of correcting the image position of the second image signal is performed. An example of a captured image obtained in Step S24 is illustrated in Fig. 12. Fig. 12 shows the example of the captured image captured using the second narrowband light having a central wavelength of 445 nm. Although not sufficiently illustrated due to constraints of illustration in Fig. 12, the contrast of the extreme surface layer blood vessels 112 in the captured image illustrated in Fig. 12 remarkably decreases as compared to that in the captured image of Fig. 11. Additionally, surface layer blood vessels 114 present at a position deeper than the extreme surface layer blood vessels 112 have a gentle decrease of the contrast as compared to that in Fig. 11.

In Step S25 of Fig. 10, the brightness correction processing unit 63 performs the light quantity correction on the acquired second image signal.

In Step S26, the computed image signal creation unit 76 performs log transformation on the second image signal subjected to the light quantity correction.

In Step S28, the computed image signal creation unit 76 performs the difference processing of creating a difference image between the second image signal log-transformed in Step S26 and the first image signal log-transformed in Step S16. In the difference processing of Step S28, a computed image signal representing the difference image is created by the computation of subtracting the first image signal from the second image signal. The computed image signal created in Step S28 is equivalent to the computed image signal ΔB that is already described.

In Step S29, the low-pass filter processing unit 77 performs low-pass filter processing on the computed image signal ΔB created in Step S28.

Then, in Step S30, the image creation unit 78 creates a blood vessel enhanced image by allocating the first image signal log-transformed in Step S16 to the brightness channel Y that is a brightness signal and allocating the computed image signal subjected to the resolution reducing processing in Step S29 to the color difference channels Cr and Cb that are color difference signals. Additionally, the image creation unit 78 performs the color conversion processing of converting the YCC image into an RGB image and creates an RGB image 100 representing the blood vessel enhanced image.

The blood vessel enhanced image created by Step S30 is displayed on the monitor 18. Additionally, the blood vessel enhanced image created by Step S30 can be saved in the storage 70. Additionally, the image signals obtained at the respective steps of Step S13, Step S23, Step S15, and Step S25 can be saved in the storage 70.

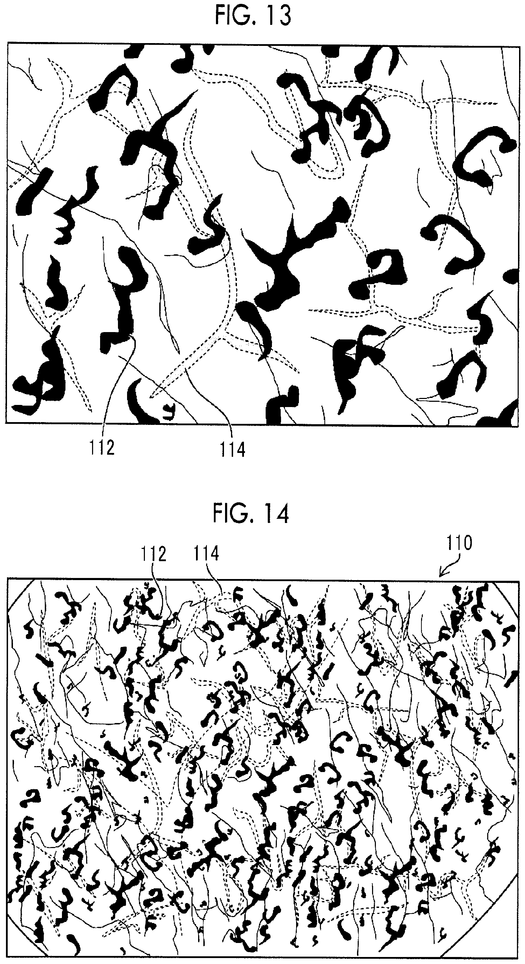

An example of an output image created through Step S30 is illustrated in Fig. 13. In Fig. 13, although not sufficiently expressed due to constraints of illustration, a color image in which the surface layer blood vessels are enhanced is obtained as the output image.

In the blood vessel enhanced image created in this way, the extreme surface layer blood vessels present in the extreme surface layer under the mucous membrane are colored and displayed in a magenta-based color, and the surface layer blood vessels in the surface layer at the position deeper than the extreme surface layer are colored and displayed in a cyan-based color. Hence, in the blood vessel enhanced image, the extreme surface layer blood vessels and the surface layer blood vessels can be distinguished from each other in colors, and particularly, and are displayed on the monitor 18 as the blood vessel visualized image that is easy to observe the extreme surface layer blood vessels.

[Specific image example 1]

Fig. 14 illustrates an example of a captured image captured using the first narrowband light having a central wavelength of 405 nm as the first illumination light. Fig. 14 is equivalent to an image example of the first image signal acquired in Step S13 of Fig. 10. The captured image illustrated in Fig. 14 is referred to as a first captured image 110. The extreme surface layer blood vessels 112 and the surface layer blood vessels 114 are projected on the first captured image 110. Fig. 11 that is previously described is equivalent to an image obtained by enlarging a portion of Fig. 14.

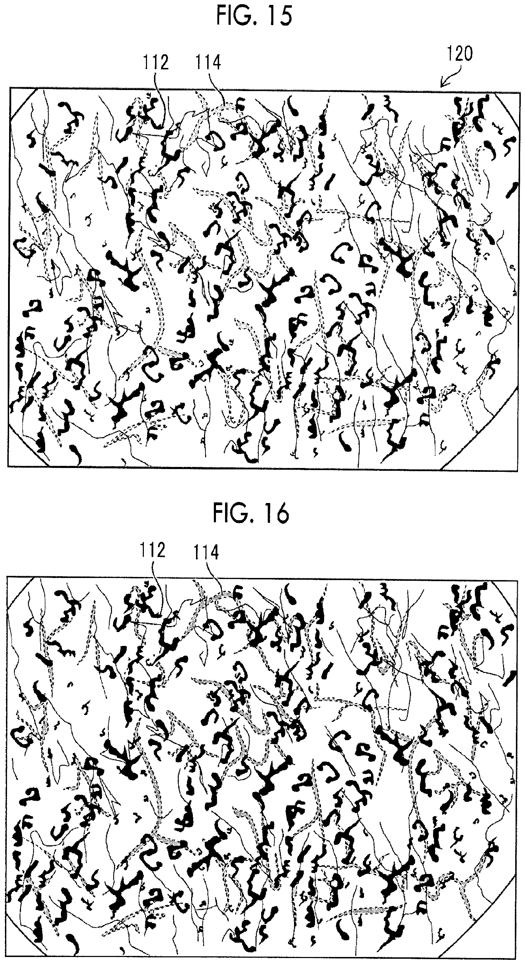

Fig. 15 illustrates an example of a captured image captured using the second narrowband light having a central wavelength of 445 nm as the second illumination light. Fig. 15 is equivalent to an image example of the second image signal acquired in Step S23 of Fig. 10. The captured image illustrated in Fig. 15 is referred to as a second captured image 120. The contrast of the extreme surface layer blood vessels 112 and the surface layer blood vessels 114 in the second captured image 120 decreases as compared to that in the first captured image 110. Fig. 12 that is previously described is equivalent to an image obtained by enlarging a portion of Fig. 15.

Fig. 16 illustrates an example of a blood vessel enhanced image created using a computed image signal resulting from the difference between the first captured image 110 and the second captured image 120. Fig. 13 that is previously described is equivalent to an image obtained by enlarging a portion of Fig. 16.

[Specific image example 2]

In the above-described Image Example 1, an example in which the first narrowband light and the second narrowband light on the short wavelength side is used as the illumination light has been described. However, blood vessels present in a deeper layer can be extracted by using two types of narrowband light on the long wavelength side. For example, image signals corresponding to respective types of narrowband light can be obtained using the narrowband light of the green wavelength range having a central wavelength of 540 nm and the narrowband light of the red wavelength range having a central wavelength of 620 nm, and a blood vessel enhanced image can be created from these image signals.

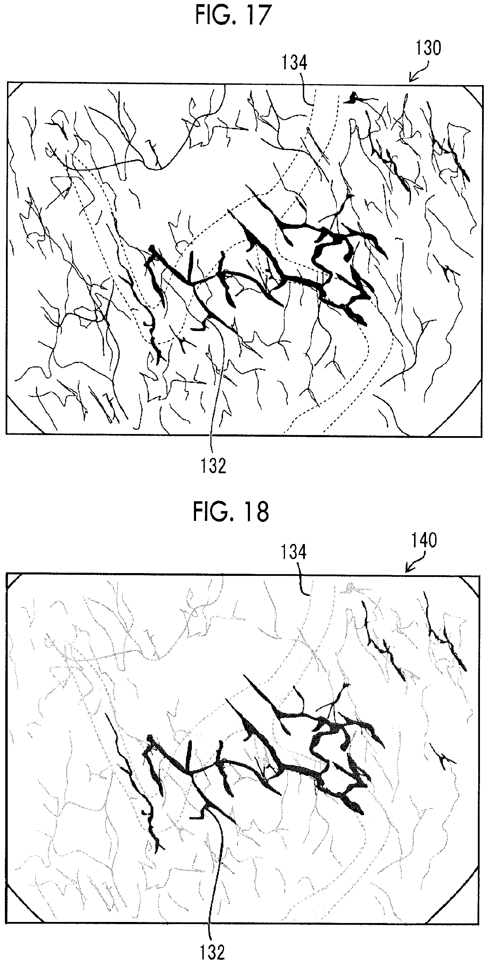

Fig. 17 illustrates an example of a captured image captured using the third narrowband light having a central wavelength of 540 nm as the first illumination light. Fig. 17 is equivalent to another image example of the first image signal acquired in Step S13 of Fig. 10. The captured image illustrated in Fig. 17 is referred to as a third captured image 130. Surface layer blood vessels 132 and middle layer blood vessels 134 are projected on the third captured image 130.

Fig. 18 illustrates an example of a captured image captured using the fourth narrowband light having a central wavelength of 620 nm as the second illumination light. Fig. 18 is equivalent to another image example of the second image signal acquired in Step S23 of Fig. 10. The captured image illustrated in Fig. 18 is referred to as a fourth captured image 140. The contrast of the surface layer blood vessels 132 and the middle layer blood vessels 134 in the fourth captured image 140 decreases as compared to that in the third captured image 130.

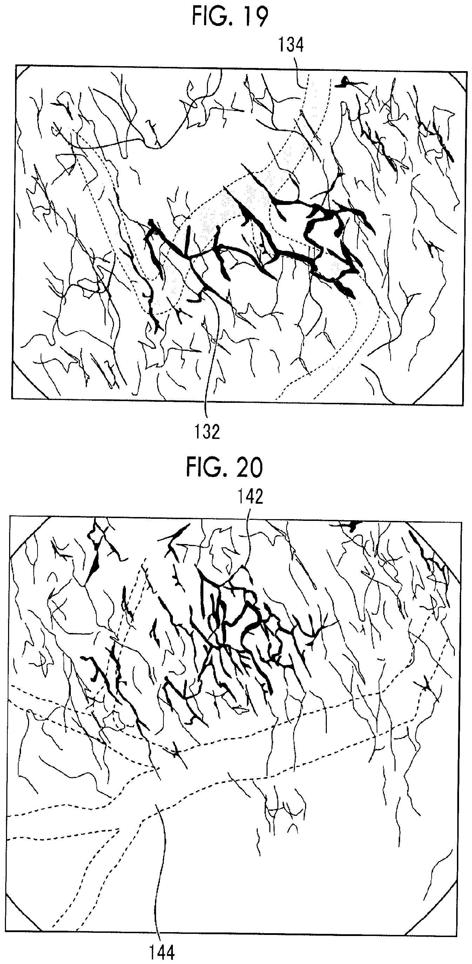

Fig. 19 illustrates an example of a blood vessel enhanced image created using a computed image signal resulting from the difference between the third captured image 130 and the fourth captured image 140.

As is clear from the comparison between Image Example 1 illustrated Figs. 14 to 16 and Image Example 2 illustrated Figs. 17 to 19, blood vessel enhanced images to be obtained vary due to differences in wavelength of the light to be used as illumination light.

[Outline of means for estimating blood vessel depth from endoscopic image]

Blood vessels at various depths can be extracted and visualized by performing imaging using a plurality of types of narrowband light having different wavelength ranges as described above. Moreover, the endoscope system 10 of the present embodiment includes the functions of estimating the value of the depth, particularly, the absolute blood vessel depth of blood vessels imaged by the endoscope 12. The absolute depth of the blood vessels is, for example, a distance in a depth direction toward the inside of the living body tissue with reference to the surface of the mucous membrane.

Fig. 20 illustrates an example of a special observation image obtained by the endoscope system 10. An example of an endoscopic image in which a blood vessel image of surface layer blood vessels 142 and the deep layer blood vessels 144 is included is illustrated in Fig. 20. As illustrated in Fig. 20, there is a clear difference clear in the shape of blood vessels between the surface layer blood vessels 142 and the deep layer blood vessels 144. In the present embodiment, the depths of blood vessels are estimated from shape patterns of blood vessels imaged by the endoscope 12 by associating the shape patterns of the blood vessels with the values of depths based on pathological information or the like.

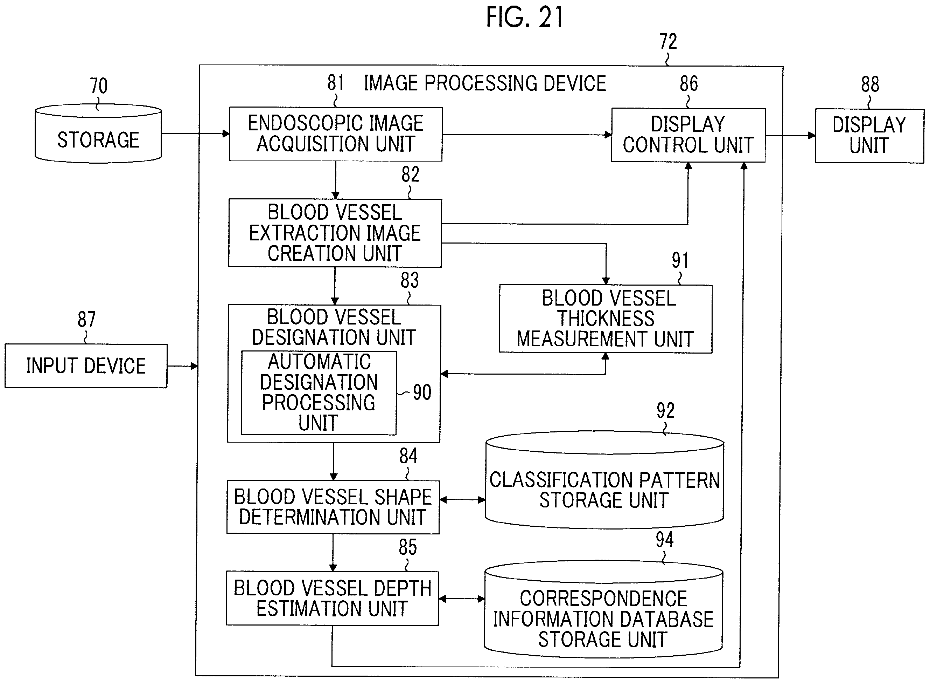

Fig. 21 is a block diagram illustrating the functions of the image processing device 72. The image processing device 72 includes an endoscopic image acquisition unit 81, a blood vessel extraction image creation unit 82, a blood vessel designation unit 83, a blood vessel shape determination unit 84, a blood vessel depth estimation unit 85, and a display control unit 86. Additionally, the image processing device 72 may include an input device 87 and a display unit 88. In addition, the console 19 described n Fig. 1 may function as the input device 87. Additionally, the monitor 18 described in Fig. 1 may function as the display unit 88.

Respective functional units of the image processing device 72 can be realized by the combination of hardware and software of a computer. Additionally, some or all of the functional units of the image processing device 72 may be realized by an integrated circuit.

The endoscopic image acquisition unit 81 acquires the endoscopic image from the storage 70. The endoscopic image means an image captured by the endoscope 12. The image signals that becomes an origin of the normal observation image, the special observation image, and the special observation image are included in the term "endoscopic image". The image signals that becomes the origin of the special observation image refer to image signals corresponding to the type of narrowband light used for the illumination light.

The endoscopic image acquired via the endoscopic image acquisition unit 81 can be displayed on the display unit 88 via the display control unit 86.

The blood vessel extraction image creation unit 82 performs the processing of creating a blood vessel extraction image from the image signals acquired via the endoscopic image acquisition unit 81. The processing of creating the blood vessel extraction image is the same as that of Step S13 to Step S16 and Step S23 to Step S30 in the image processing of creating the blood vessel enhanced image described in the flowchart of Fig. 10. The blood vessel extraction image can be read as the blood vessel enhanced image.

For example, in a case where the endoscopic image acquisition unit 81 acquires the image signals obtained in the respective steps of Step S13 and Step S23 of Fig. 10 from storage 70, the blood vessel extraction image creation unit 82 performs processing of Step S15, Step S16, and Step S25 to Step S30 of Fig. 10.

Additionally, in a case where the endoscopic image acquisition unit 81 acquires the image signals obtained in the respective steps of Step S15 and Step S23 of Fig. 10 from storage 70, the blood vessel extraction image creation unit 82 performs processing of Step S16 and Step S26 to Step S30 of Fig. 10.

In addition, in a case where the endoscopic image acquisition unit 81 acquires the blood vessel enhanced image created through the processing of Step S30 of Fig. 10 from the storage 70, the processing by the blood vessel extraction image creation unit 82 is omitted. The blood vessel extraction image created by the blood vessel extraction image creation unit 82 can be displayed on the display unit 88 via the display control unit 86.

The blood vessel designation unit 83 designates blood vessels of interest that serve as a target for estimating the blood vessel depth out of the blood vessels included in the endoscopic image. As one of the methods of designating the blood vessels of interest, a user designates blood vessels of interest for estimating the depth, using a user interface, for example on the endoscopic image displayed on the display unit 88. The input device 87 includes a pointing device, a keyboard, and the like that are used for the designation of the blood vessels of interest, or the like. The input device 87 may be a touch panel configured integrally with the display screen of the display unit 88. The blood vessels of interest are not limited to one, and a plurality of blood vessels or a blood vessel group may be designated as the blood vessels of interest. All the blood vessels projected on the endoscopic image may be designated as the blood vessels of interest. For example, the blood vessels of interest may be directly selected by a method of causing the user to click a blood vessel portion on the image or moving a cursor along blood vessels or the like, or a specific region of interest may be designated on the image and blood vessels included within the region may be selected as the blood vessels of interest.

The blood vessel designation unit 83 may include an automatic designation processing unit 90. The automatic designation processing unit 90 performs the processing of automatically determining the blood vessels of interest from the endoscopic image. The automatic designation processing unit 90 performs the processing of automatically designating some or all of the blood vessels projected on the endoscopic image as the blood vessels of interest. As one of the methods of the automatic designation by the automatic designation processing unit 90, the blood vessels of interest are automatically designated, for example, in accordance with the type of illumination light radiated during imaging. The automatic designation processing unit 90 performs the processing of switching the blood vessels of interest in accordance with the wavelength of illumination light. As specific examples, thin blood vessels within the endoscopic image are designated as the blood vessels of interest in the case of an endoscopic image captured by radiating the short-wavelength illumination light, while relatively thick blood vessels within the endoscopic image are designated as the blood vessels of interest in the case of an endoscopic image captured by radiating the illumination light on the long wavelength side.

The automatic designation processing unit 90 designates blood vessels thinner than a regular blood vessel thickness, among blood vessels extracted from a captured image captured using illumination light having a wavelength range on a relatively short wavelength side, as the blood vessels of interest. Additionally, the automatic designation processing unit 90 designates blood vessels thicker than a regular blood vessel thickness, among blood vessels extracted from a captured image captured using illumination light having a wavelength range on a relatively long wavelength side, as the blood vessels of interest. The "regular blood vessel thickness" used as a threshold value in a case where the thin blood vessels are designated and the "regular blood vessel thickness" used as a threshold value in a case where the thick blood vessels are designated can be set to suitable values, respectively, in advance.

The image processing device 72 has a blood vessel thickness measurement unit 91 that measures the thickness of the blood vessels from the endoscopic image. The blood vessel thickness measurement unit 91 may measure the thickness of the blood vessels from the blood vessel extraction image created by the blood vessel extraction image creation unit 82, or may measure the thickness of the blood vessels from the image acquired from the endoscopic image acquisition unit 81. The automatic designation processing unit 90 can designate the blood vessels of interest, utilizing thickness information on the blood vessels obtained by the blood vessel thickness measurement unit 91. It is also possible to adopt a form in which the automatic designation processing unit 90 includes the functions of the blood vessel thickness measurement unit 91.

Additionally, as another configuration example of the automatic designation processing unit 90, the type of blood vessels having the highest contrast on the endoscopic image may be designated the blood vessels of interest. Although the blood vessel designation unit 83 just has to have at least one of means for allowing the user to manually select the blood vessels of interest or means for automatically determining the blood vessels of interest, a configuration in which both these means are provided and are appropriately used in accordance with situations is preferable.

In addition, in a case where all the blood vessels projected on the endoscopic image are automatically handled as the blood vessels of interest, the processing of designating the blood vessels of interest may be omitted. That is, in the case of in the configuration in which all the blood vessels included in the endoscopic image are automatically handled as the blood vessels of interest, a form in which the blood vessel designation unit 83 is omitted may be adopted. Of course, in the case of the configuration in which all the blood vessels projected on the endoscopic image are automatically handled as the blood vessels of interest, it may be understood that the automatic designation processing unit 90 designates all the blood vessels included in the endoscopic image as the blood vessels of interest.

The blood vessel shape determination unit 84 determines the shape pattern of a blood vessel image of the blood vessels of interest related to the designation of the blood vessel designation unit 83. The shape patterns of the blood vessels are classified, for example, in accordance with the types of respective blood vessels, such as intra-epithelial papillary capillary loops (IPCL) and palisading blood vessels.

Shape patterns of various blood vessels can be discriminated in accordance with the classification patterns that are determined in advance. The classification patterns are shape patterns for reference that are prepared for each type of blood vessels. The blood vessel shape determination unit 84 extracts the portion of the blood vessels of interest in the blood vessel extraction image, and checks the blood vessel shape of the portion with the classification patterns, thereby searching for which classification pattern is applied, and discriminating a shape pattern of the blood vessels of interest.

Additionally, since a blood vessel image of a surface layer portion is complicated as compared to a blood vessel image of the middle-depth layer, the blood vessel image may be determined using feature amounts, such as the number of branches and the number of loops in the shape patterns of the blood vessel image, instead of or in combination with the above-described pattern recognition processing by the classification patterns. For example, in a case where the number of branches and the number of loops per unit area of the blood vessels of interest are respectively more than regular numbers, the blood vessels of interest can be determined to be the surface layer blood vessels. In addition, the number of feature amounts to be used for the determination of the blood vessel shape patterns may be one, or a combination of two or more types of feature amounts may be adopted.

The image processing device 72 of the present embodiment has a classification pattern storage unit 92. Classification pattern database, which is a data aggregate of the classification patterns prepared in advance, is stored in the classification pattern storage unit 92. Additionally, data on the feature amounts, such as the number of branches and the number of loops, in the blood vessel image, may be stored in the classification pattern storage unit 92.

The blood vessel shape determination unit 84 extracts the blood vessel portion of the blood vessels of interest of the blood vessel extraction image, and discriminates a shape pattern of the blood vessels of interest, using the data stored in the classification pattern storage unit 92. The shape patterns may include information on the thickness of the blood vessels. The blood vessel shape determination unit 84 can acquire the information on the thickness of the blood vessels of interest from the blood vessel thickness measurement unit 91. In addition, it is also possible to adopt a form in which the blood vessel shape determination unit 84 includes the functions of the blood vessel thickness measurement unit 91.

The thickness (vessel diameter) of the blood vessels is a distance between a blood vessel and a boundary line of the mucous membrane, and is counted, for example, by counting the number of pixels along the lateral direction of an extracted blood vessel through the blood vessel from an edge of the blood vessel. The thickness of the blood vessels can be expressed by the number of pixels. However, in a case where an imaging distance, a zoom magnification factor, and the like in a case where the endoscopic image is captured are known, the thickness can be converted into the unit of length, such as "micrometer [$\mu$m]" if necessary.

Here, in a case where the thickness of the blood vessels are measured from the blood vessel extraction image, it is also possible to consider that the pixel values fluctuate under the influence of the unevenness of the light source, the quantity fluctuation of the illumination light, or the like, and accurate thickness cannot be measured. Hence, it is more preferable that the shape patterns are patterns that do not include the information on the thickness of the blood vessels. For example, it is preferable that the shape patterns are patterns of line drawings obtained by extracting the centerlines (also referred to as central lines) of the blood vessels, or patterns obtained by catching geometric features, such as loop-like patterns and branch-like patterns, or the like. The blood vessel shape may be determined by combining the shape patterns that do not include the information on thickness, and the information on thickness with each other.

The blood vessel depth estimation unit 85 estimates a blood vessel depth from the shape pattern of the blood vessels determined by the blood vessel shape determination unit 84. The blood vessel depth estimation unit 85 of the present embodiment estimates the blood vessel depth of the blood vessels of interest, utilizing correspondence information in which the blood vessel shape and the depth are associated with each other. Correspondence information, which defines a correspondence relationship between the blood vessel shape and the depth of various blood vessels, is prepared in advance on the basis of the pathological information or the like.

As the type of blood vessels, there are mainly the surface layer blood vessels distributed in the surface layer of the living body tissue and the middle-depth layer blood vessels located below the surface layer. Blood vessel images depicted in the endoscopic image are different from each other in the surface layer blood vessels and the middle-depth layer blood vessels (refer to Fig. 20).

For example, in the case of the esophagus, thin blood vessels, such as the IPCL, can be determined as the surface blood vessels, and the palisading blood vessels can be determined as the deep layer blood vessels. It is known that the surface layer blood vessels have a depth of approximately 50 $\mu$m and the deep layer blood vessels have a depth of approximately 200 $\mu$m. The absolute depth of the blood vessels of interest can be known by utilizing such correspondence information.

The image processing device 72 of the present embodiment has a correspondence information database storage unit 94. A correspondence information database, which is an aggregate of the correspondence information that defines the correspondence relationship between the blood vessel shape and the depth of the various blood vessels, is stored in the correspondence information database storage unit 94. The correspondence information is prepared in accordance with respective regions of the living body tissue used as the observation target, and appropriate correspondence information is referred to in accordance with a region to be observed that is the observation target.

The blood vessel depth estimation unit 85 estimates the blood vessel depth by checking the shape pattern determined by the blood vessel shape determination unit 84 with the correspondence information database. For example, the numerical values of the absolute depth can be determined on the conditions of "50 $\mu$m" in a case where the blood vessels of interest are the surface layer blood vessels and "200 $\mu$m" in a case of the blood vessels of interest are the deep layer blood vessels.

Additionally, the blood vessel depth estimation unit 85 may estimate the value of the depth of the middle layer blood vessels to a value between the value of the depth of the surface layer blood vessels and the value of the depth of the deep layer blood vessels, in a case where the blood vessels of interest are the middle-depth blood vessels applicable to neither the surface layer blood vessels nor the deep layer blood vessels, for example, in the case of blood vessels or the like that are thicker than the surface layer blood vessels and thinner than the deep layer blood vessels. In this way, the blood vessel depth may be estimated by combining the information on the shape patterns and the thickness of the blood vessels. In this case, the blood vessel depth estimation unit 85 may acquire the information on the thickness of the blood vessels from the blood vessel thickness measurement unit 91. In addition, it is also possible to adopt a form in which the blood vessel depth estimation unit 85 includes the functions of the blood vessel thickness measurement unit 91.

The information on the blood vessel depth estimated by the blood vessel depth estimation unit 85 is sent to the display control unit 86. The display control unit 86 controls the display of the display unit 88. The display control unit 86 performs the display control of displaying the information on the blood vessel depth estimated by the blood vessel depth estimation unit 85 together with the image in which the blood vessels of interest are included, on the display unit 88. Additionally, the display control unit 86 can display either the endoscopic image acquired via the endoscopic image acquisition unit 81 or the blood vessel extraction image created by the blood vessel extraction image creation unit 82 or a combination thereof, on the display unit 88. The display control unit 86 controls the display contents of the display unit 88 in accordance with an instruction for selection or switching of a display image by the input device 87 or an instruction of switching of display modes, such as one screen display and multi-screen display. A combination of the display control unit 86 and the display unit 88 is equivalent to one form of an "information presentation unit".

[Processing flow regarding estimation of blood vessel depth].

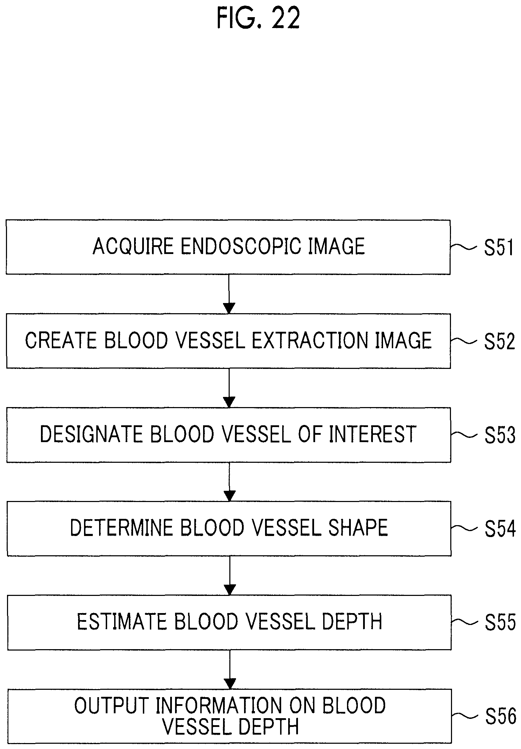

Fig. 22 is a flowchart illustrating a flow of processing of estimating the blood vessel depth in the endoscope system 10 of the present embodiment. The operation illustrated in Fig. 22 can be understood as a method of operating the image processing device 72. Additionally, the operation illustrated in Fig. 22 can be understood as a method of operating the endoscope system 10.

In Step S51, the endoscope system 10 acquires the endoscopic image. Step S51 is equivalent to one form of an "image signal acquisition step". A step of acquiring the image signals from the endoscope 12 by the image signal acquisition unit 53 of the processor device 16 is equivalent to one form of an endoscopic image acquisition step of Step S51. Additionally, a step of acquiring the endoscopic image from the storage 70 by the endoscopic image acquisition unit 81 of the image processing device 72 is equivalent to one form of the endoscopic image acquisition step of Step S51.

In Step S52, the endoscope system 10 creates the blood vessel extraction image on the basis of the endoscopic image acquired in Step S51. The blood vessel extraction image is an image created through the processing of extracting or enhancing the blood vessel image. The concept of reorganization processing that is distinguishable from others or the concept of differentiation processing that is distinguishable from others is also included in the "extraction". The special observation image that is the blood vessel enhanced image is equivalent to one form of the blood vessel extraction image. Additionally, the blood vessel extraction image may be a synthesized image synthesized on the basis of a plurality of blood vessel enhanced images, or may be an image obtained by synthesizing the normal observation image and the special observation image.

A step of creating the blood vessel enhanced image by the signal processing unit 60 of the processor device 16 is equivalent to one form of a blood vessel extraction image creation step of Step S52. Additionally, a step of creating the blood vessel extraction image by the blood vessel extraction image creation unit 82 of the image processing device 72 is equivalent to one form of the blood vessel extraction image creation step of Step S52.

In Step S53, the blood vessel designation unit 83 designates the blood vessels of interest. As already described, the blood vessels of interest may be designated on the basis of the operation of the user, or may be automatically designated from the endoscopic image. In addition, in a case where all the blood vessels within the endoscopic image are automatically used as the blood vessels of interest, a blood vessel designation step of Step S53 may be omitted.

In Step S54, the blood vessel shape determination unit 84 determines the blood vessel shape of the blood vessels of interest. The blood vessel shape determination unit 84 determines the shape pattern of the blood vessels of interest on the basis of the image signals acquired in Step S51. Step S54 is equivalent to one form of a "blood vessel shape determination step".

In Step S55, the blood vessel depth estimation unit 85 estimates the blood vessel depth of the blood vessels of interest on the basis of the shape pattern determined in Step S54. Step S55 is equivalent to one form of a "blood vessel depth estimation step".

In Step S56, the blood vessel depth estimation unit 85 outputs the information on the blood vessel depth estimated in Step S55. As an example of a specific output form, the information on the blood vessel depth estimated by the blood vessel depth estimation unit 85 together with the image including the blood vessels of interest is displayed on the display unit 88.

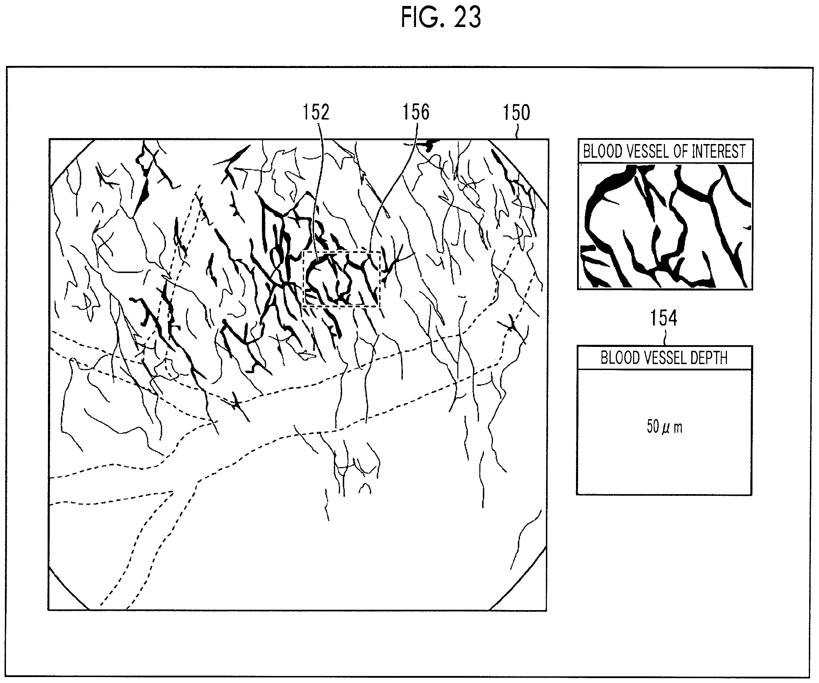

Fig. 23 illustrates an example of a display screen of the display unit 88. In a case where blood vessels of interest 152 are designated in an endoscopic image 150 from which blood vessels are extracted, the blood vessel depth of the blood vessels of interest 152 is displayed on a blood vessel depth display window 154 within the screen. A blood vessel designation frame 156 is an operation assisting mark indicating a region where the blood vessels of interest 152 are included, on the endoscopic image 150. By operating the input device 87, the user can move the blood vessel designation frame 156 on the endoscopic image 150. Selection and change of the blood vessels of interest are allowed by the blood vessel designation frame 156.

[Operational effects of first embodiment]

According to the first embodiment, the absolute blood vessel depth can be estimated without using the information on the scattering coefficient of the observation target. Additionally, information useful for diagnosis can be provided by displaying the information on the estimated blood vessel depth together with an endoscopic image on the display unit 88.

[Second embodiment]

Although a configuration in which the endoscope system 10 saves the endoscopic image in the storage 70 and the image processing device 72 acquires the endoscopic image from the storage 70 to estimate the blood vessel depth has been described in the first embodiment, the endoscope system 10 may execute the processing of estimating the blood vessel depth substantially in real time while observing the observation target.

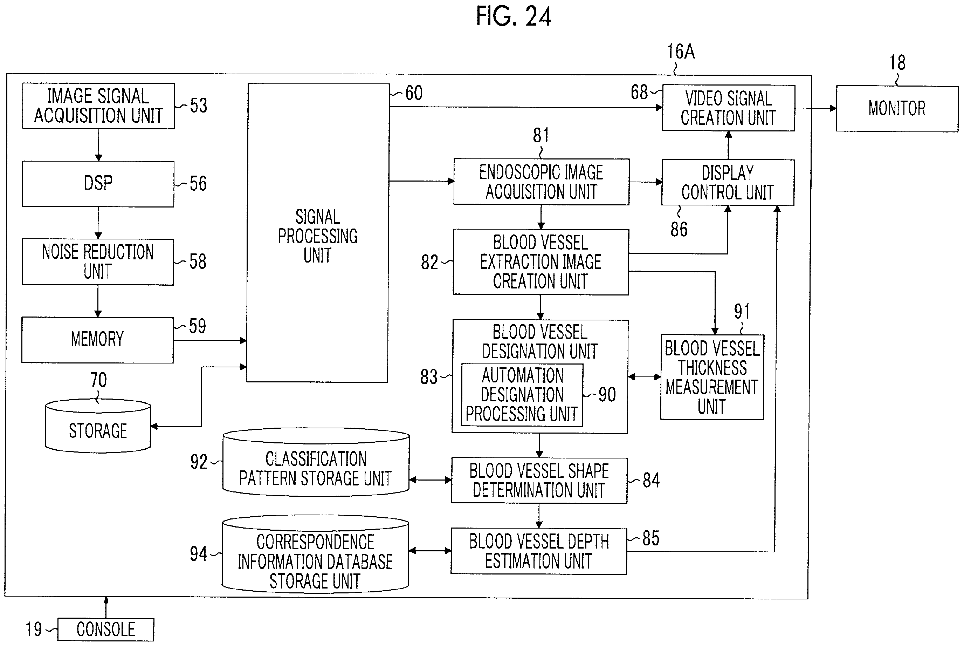

Fig. 24 is a block diagram illustrating the functions of a processor device of an endoscope system related to a second embodiment. In Fig. 24, elements that are the same or similar to the configuration described in Figs. 2 and 21 will be designated by the same reference signs, and the description thereof will be omitted. In Fig. 24, illustration of the endoscope 12 and the light source device 14 that are described in Fig. 2 is omitted. However, in the endoscope system of the second embodiment, the endoscope 12 and the light source device 14 may adopt the same components as those of the first embodiment. A processor device 16A illustrated in Fig. 24 can be used instead of the processor device 16 illustrated in Fig. 2. The processor device 16A includes the functions of the image processing device 72 described in Fig. 2. Additionally, the processor device 16A includes a storage unit that plays a role of storage 70, inside the device.

The console 19 plays a role of the input device 87 described in Fig. 21. The monitor 18 plays a role of the display unit 88 described in Fig. 21. Hence, a configuration in which the input device 87 and the display unit 88 are omitted can be adopted in the second embodiment.

The endoscopic image acquisition unit 81 can acquire the endoscopic image from the signal processing unit 60 without using the storage 70. In a case where the signal processing unit 60 creates the blood vessel enhanced image in accordance with the flowchart of Fig. 10 in the special observation mode, the processing performed by the blood vessel extraction image creation unit 82 may be omitted, and the blood vessel designation unit 83 may acquire the blood vessel enhanced image from the signal processing unit 60. Additionally, in the case of a form in which the blood vessel designation unit 83 is omitted, the blood vessel shape determination unit 84 may acquire the blood vessel enhanced image from the signal processing unit 60.

The blood vessel thickness measurement unit 91 may measure the thickness of the blood vessels from the endoscopic image created by the signal processing unit 60. The processor device 16A in the second embodiment is equivalent to one form of the "image processing device".

According to the second embodiment, the blood vessel depth of the blood vessels of interest can be estimated similarly to the first embodiment. Additionally, according to the second embodiment, it is possible to estimate the blood vessel depth, using endoscopic images serially created in the special observation mode, irrespective of the presence or absence of an instruction from the still image acquisition instruction unit 13c.

The implementation of the invention is not limited to the above-described first embodiment and second embodiment, and various forms may be adopted. Hereinafter, some modification examples regarding the embodiments will be disclosed.

[Modification example 1]

The classification pattern storage unit 92 or the correspondence information database storage unit 94 described in Fig. 23 or both of them may be provided in an external device separate from the image processing device 72. For example, the classification pattern storage unit 92 or the correspondence information database storage unit 94, or both of them, may be mounted on a server communicably connected to the image processing device 72.

[Modification example 2]

The information on the blood vessel depth estimated by the blood vessel depth estimation unit 85 may be saved in the storage 70. For example, a configuration in which image data obtained by combining the information on the blood vessel depth with the endoscopic image of the blood vessels of interest can be saved in the storage 70 may be adopted.

[Modification example 3]

The control of supplying the information on the blood vessel depth estimated by the blood vessel depth estimation unit 85 to the light source control unit 22 to select the illumination light suitable for the observation of the blood vessels of interest may be performed.

[Modification example 4]

The image to be displayed on the display unit 88 in a case where the user manually designates the blood vessels of interest is not limited to the special observation image, and may be the normal observation image or may be the synthesized image obtained by synthesizing the normal observation image and the special observation image.

[Modification example 5]

Although an example in which the blood vessel enhanced image is created from the images captured using the two types of illumination light having different wavelengths, respectively, is described in the flowchart of Fig. 10, the blood vessels may be extracted from a plurality of images captured using three or more types of illumination light having different wavelengths, respectively.

[Modification example 6]

In the above embodiment, the cut-off frequency of the LPF to be used in the low-pass filter processing unit 77 is set in advance. However, it is preferable to make the cut-off frequency of the LPF variable and dynamically set the cut-off frequency of the LPF. For example, information on the alignment accuracy of the first image signal and the second image signal is input from the alignment processing unit 62 to the low-pass filter processing unit 77. Then, the low-pass filter processing unit 77 changes the cut-off frequency of the LPF, that is, the intensity of the resolution reducing processing in accordance with the alignment accuracy of the first image signal and the second image signal. Specifically, as the alignment accuracy is higher, the cut-off frequency of the LPF may be set to a higher frequency to make the intensity of the resolution reducing processing smaller, and as the alignment accuracy is lower, the cut-off frequency of the LPF may be set to a lower frequency to make the intensity of the resolution reducing processing larger.

In addition, in a case where the blood vessel enhanced image is displayed or saved as a still image, it is preferable the cut-off frequency of the LPF is set to be at least within a range where at least a frequency of 1/8 or less of the Nyquist frequency is left, with the resolution of the blood vessel enhanced image to be created as a reference.

[Modification example 7]

In Modification Example 6, the low-pass filter processing unit 77 regulates the intensity of the resolution reducing processing in accordance with the accuracy of alignment processing of the alignment processing unit 62. However, contrary to this, the alignment processing unit 62 may regulate the accuracy of alignment processing in accordance with the intensity of the resolution reducing processing performed by the low-pass filter processing unit 77. In this case, the alignment processing unit 62 set the alignment accuracy of the first image signal and the second image signal to a higher value as the cut-off frequency of the LPF is set to be larger and the intensity of the resolution reducing processing is set to be smaller.

In a case where the accuracy of alignment processing of the first image signal and the second image signal performed by the alignment processing unit 62 is made variable and the still image of the blood vessel enhanced image is displayed or saved, and in a case where a moving image of the blood vessel enhanced image is displayed, it is preferable to change the accuracy of alignment processing. For example, in a case where the moving image of the blood vessel enhanced image is displayed on the monitor 18, the alignment processing unit 62 aligns the first image signal and the second image signal with each other with a first accuracy lower than that in a case where the still image of the blood vessel enhanced image is displayed (or saved) on the monitor 18. Contrary to this, in a case where the still image of the blood vessel enhanced image is displayed on the monitor 18, the alignment processing unit 62 performs the alignment with a second accuracy higher than that in a case where the moving image of the blood vessel enhanced image is displayed on the monitor 18. In this way, at the time of the display of the moving image, the blood vessel enhanced image can be generated at a high speed within a range where the color deviation is not conspicuous, and at the time of the acquisition of a still image with a conspicuous color deviation, the blood vessel enhanced image in which the color deviation is further suppressed can be created.

[Modification example 8]

The resolution reducing processing can also be performed by reducing the computed image signal ΔB and then enlarging the image signal to its original size instead of the low-pass filter processing unit 77. In this way, in a case where the computed image signal ΔB is reduced and enlarged to reduce the resolution, it is preferable to adopt a reduction method with less aliasing at the time of reduction of the computed image signal ΔB. For example, the computed image signal ΔB can be reduced in resolution after being reduced by the area average method and then enlarged by cubic spline interpolation.

[Modification example 9]

The invention can also be applied to a capsule endoscope system using a capsule endoscope instead of the endoscope 12 described in Fig. 1.

Although the embodiments and the modification examples of the invention have been described above, the invention is not limited to these embodiments and modification examples, and the invention can be modified in various forms without departing from the concept of the invention.

Explanation of References

10: endoscope system
12: endoscope
12a: insertion part
12b: operating part
12c: bending part
12d: distal end part
12e: angle knob
13a: mode changeover switch
13b: zooming operation part
13c: still image acquisition instruction unit
14: light source device
16, 16A: processor device
18: monitor
19: console
20: light source
22: light source control unit
23a: V-LED
23b: B-LED
23c: G-LED
23d: R-LED
30a: illumination optical system
30b: imaging optical system
41: light guide
45: illumination lens
46: objective lens
47: zoom lens
48: imaging sensor
51: AFE circuit
52: AD converter
53: image signal acquisition unit
56: DSP
58: noise reduction unit
59: memory
60: signal processing unit
61: image processing switching unit
62: alignment processing unit
63: brightness correction processing unit
66: normal observation image processing unit
67: special observation image processing unit
68: video signal creation unit
70: storage

72: image processing device
76: computed image signal creation unit
77: low-pass filter processing unit
78: image creation unit
81: endoscopic image acquisition unit
82: blood vessel extraction image creation unit
83: blood vessel designation unit
84: blood vessel shape determination unit
85: blood vessel depth estimation unit
86: display control unit
87: input device
88: display unit
90: automatic designation processing unit
91: blood vessel thickness measurement unit
92: classification pattern storage unit
94: correspondence information database storage unit
100: RGB image
110: first captured image
112: extreme surface layer blood vessel
114, 132, 142: surface layer blood vessel
120: second captured image
130: third captured image
134: middle layer blood vessel
140: fourth captured image
144: deep layer blood vessels
150: endoscopic image
152: blood vessels of interest
154: display window
156: blood vessel designation frame
S11 to S30: image processing step of special observation mode
S51 to S56: step of processing of estimating blood vessel depth

**Claims**

1. An endoscope system comprising:

   a light source unit that generates a plurality of types of illumination light having different wavelength ranges;
   an imaging sensor that images an observation target irradiated with any one of the plurality of types of illumination light;
   a blood vessel shape determination unit that, on the basis of an image signal obtained from the imaging sensor, determines a shape pattern of blood vessels of interest that are some or all of blood vessels included in a captured image of the observation target; and
   a blood vessel depth estimation unit that estimates a blood vessel depth of the blood vessels of interest on the basis of the shape pattern.

2. The endoscope system according to claim 1,

   wherein the blood vessel depth estimation unit estimates the blood vessel depth of the blood vessels of interest, utilizing correspondence information in which a blood vessel shape and a blood vessel depth are associated with each other.

3. The endoscope system according to claim 2, further comprising:

   a database storage unit that stores a database regarding the correspondence information.

4. The endoscope system according to claim 3,

wherein the correspondence information is prepared in accordance with respective regions of a living body tissue used as the observation target, and appropriate correspondence information is referred to in accordance with a region to be observed that is the observation target.

5. The endoscope system according to any one of claims 1 to 4, further comprising:

a blood vessel thickness measurement unit that measures a thickness of the blood vessels from the captured image of the observation target acquired via the imaging sensor,
wherein the blood vessel depth estimation unit estimates the blood vessel depth on the basis of information on the shape pattern of the blood vessels of interest and the thickness of the blood vessels of interest obtained by the blood vessel thickness measurement unit.

6. The endoscope system according to any one of claims 1 to 5, further comprising:

a blood vessel designation unit that designates the blood vessels of interest from the captured image of the observation target acquired via the imaging sensor.

7. The endoscope system according to claim 6, further comprising:

a blood vessel extraction image creation unit that creates a blood vessel extraction image obtained by extracting a blood vessel portion from the image signal obtained from the imaging sensor,
wherein the blood vessel designation unit designates the blood vessels of interest from the blood vessel extraction image serving as the captured image.

8. The endoscope system according to claim 6 or 7, further comprising:

a display unit that displays the image created on the basis of the image signal obtained from the imaging sensor,
wherein the blood vessel designation unit includes an operating part for performing an operation in which a user designates the blood vessels of interest on the image displayed on the display unit.

9. The endoscope system according to any one of claims 6 to 8,

wherein the blood vessel designation unit includes an automatic designation processing unit that automatically designates the blood vessels of interest.

10. The endoscope system according to claim 9,

wherein the blood vessel designation unit automatically designates the blood vessels of interest in accordance with a wavelength range of the illumination light radiated to the observation target in a case where the observation target is imaged.

11. The endoscope system according to claim 10,

wherein the blood vessel designation unit designates a blood vessel thinner than a regular blood vessel thickness as the blood vessels of interest in a case where the observation target is imaged using the illumination light having a wavelength range on a relatively short wavelength side among the plurality of types of illumination light.

12. The endoscope system according to claim 10 or 11,

wherein the blood vessel designation unit designates a blood vessel thicker than a regular blood vessel thickness as the blood vessels of interest in a case where the observation target is imaged using the illumination light having a wavelength range on a relatively long wavelength side among the plurality of types of illumination light.

13. The endoscope system according to claim 9,

wherein the blood vessel designation unit designates a type of a blood vessel having the highest contrast among types of the blood vessels included in the captured image, as the blood vessels of interest.

**14.** The endoscope system according to any one of claims 1 to 13,

wherein the blood vessel shape determination unit determines the shape pattern of the blood vessels of interest on the basis of information on classification patterns of a blood vessel shape determined in advance in accordance with types of the blood vessels.

**15.** The endoscope system according to any one of claims 1 to 14,

wherein the blood vessel shape determination unit determines the shape pattern, using at least one feature amount of the number of branches and the number of loops of the blood vessels.

**16.** The endoscope system according to any one of claims 1 to 15, further comprising:

an information presentation unit that presents information on the blood vessel depth estimated by the blood vessel depth estimation unit together with the image in which the blood vessels of interest are included.

**17.** An image processing device comprising:

an image signal acquisition unit that acquires an image signal that is obtained by irradiating an observation target with a plurality of types of illumination light having different wavelength ranges and by imaging the observation target, using an imaging sensor, under the irradiation of the respective types of illumination light;
a blood vessel shape determination unit that determines a shape pattern of blood vessels of interest that are some or all of blood vessels included in a captured image of the observation target on the basis of the image signal acquired by the image signal acquisition unit; and
a blood vessel depth estimation unit that estimates a blood vessel depth of the blood vessels of interest on the basis of the shape pattern.

**18.** A method of operating an image processing device, comprising:

an image signal acquisition step of acquiring an image signal that is obtained by irradiating an observation target with a plurality of types of illumination light having different wavelength ranges and by imaging the observation target, using an imaging sensor, under the irradiation of the respective types of illumination light;
a blood vessel shape determination step of determining a shape pattern of blood vessels of interest that are some or all of blood vessels included in a captured image of the observation target on the basis of the image signal acquired by the image signal acquisition step; and
a blood vessel depth estimation step of estimating a blood vessel depth of the blood vessels of interest on the basis of the shape pattern.

FIG. 1

EP 3 446 617 A1

FIG. 2

## FIG. 3

## FIG. 4

## FIG. 5

## FIG. 6

## FIG. 7

67

**SPECIAL OBSERVATION IMAGE PROCESSING UNIT**

| COMPUTED IMAGE SIGNAL CREATION UNIT | 76 |

| LOW-PASS FILTER PROCESSING UNIT | 77 |

| IMAGE CREATION UNIT | 78 |

## FIG. 8

$A_s$

$A_d$

CONTRAST OF BLOOD VESSEL

VIOLET LIGHT V

BLUE LIGHT B

SURFACE LAYER

DEPTH OF BLOOD VESSEL

## FIG. 9

B1 ——————————————————→ Y

ΔB ———— $\alpha \times \Delta B$ ——→ Cb

ΔB ———— $\beta \times \Delta B$ ——→ Cr

## FIG. 10

| GENERATE ILLUMINATION LIGHT WITH FIRST WAVELENGTH RANGE | S11 | GENERATE ILLUMINATION LIGHT WITH SECOND WAVELENGTH RANGE | S21 |

| IMAGE OBSERVATION TARGET | S12 | IMAGE OBSERVATION TARGET | S22 |

| ACQUIRE IMAGE SIGNAL | S13 | ACQUIRE IMAGE SIGNAL | S23 |

| | | ALIGNMENT PROCESSING | S24 |

| LIGHT QUANTITY CORRECTION | S15 | LIGHT QUANTITY CORRECTION | S25 |

| LOG TRANSFORMATION | S16 | LOG TRANSFORMATION | S26 |

+

– DIFFERENCE PROCESSING — S28

LPF PROCESSING — S29

BLOOD VESSEL ENHANCED IMAGE CREATION (YCC→RGB) — S30

RGB IMAGE — 100

## FIG. 11

112          114

## FIG. 12

112          114

## FIG. 13

112          114

## FIG. 14

112    114                                    110

## FIG. 15

112　114　　　　　　　　　　　　120

## FIG. 16

112　114

## FIG. 17

## FIG. 18

## FIG. 19

134

132

## FIG. 20

142

144

FIG. 21

72 IMAGE PROCESSING DEVICE

88 DISPLAY UNIT

86 DISPLAY CONTROL UNIT

81 ENDOSCOPIC IMAGE ACQUISITION UNIT

82 BLOOD VESSEL EXTRACTION IMAGE CREATION UNIT

83 BLOOD VESSEL DESIGNATION UNIT

90 AUTOMATIC DESIGNATION PROCESSING UNIT

91 BLOOD VESSEL THICKNESS MEASUREMENT UNIT

84 BLOOD VESSEL SHAPE DETERMINATION UNIT

85 BLOOD VESSEL DEPTH ESTIMATION UNIT

92 CLASSIFICATION PATTERN STORAGE UNIT

94 CORRESPONDENCE INFORMATION DATABASE STORAGE UNIT

70 STORAGE

87 INPUT DEVICE

# FIG. 22

| | |
|---|---|
| ACQUIRE ENDOSCOPIC IMAGE | S51 |
| CREATE BLOOD VESSEL EXTRACTION IMAGE | S52 |
| DESIGNATE BLOOD VESSEL OF INTEREST | S53 |
| DETERMINE BLOOD VESSEL SHAPE | S54 |
| ESTIMATE BLOOD VESSEL DEPTH | S55 |
| OUTPUT INFORMATION ON BLOOD VESSEL DEPTH | S56 |

FIG. 23

EP 3 446 617 A1

## FIG. 24

IMAGE SIGNAL ACQUISITION UNIT ~53

DSP ~56

NOISE REDUCTION UNIT ~58

MEMORY ~59

STORAGE ~70

CONSOLE ~19

SIGNAL PROCESSING UNIT ~60

ENDOSCOPIC IMAGE ACQUISITION UNIT ~81

BLOOD VESSEL EXTRACTION IMAGE CREATION UNIT ~82

BLOOD VESSEL DESIGNATION UNIT ~83
AUTOMATION DESIGNATION PROCESSING UNIT ~90

CLASSIFICATION PATTERN STORAGE UNIT ~92

BLOOD VESSEL SHAPE DETERMINATION UNIT ~84

CORRESPONDENCE INFORMATION DATABASE STORAGE UNIT ~94

BLOOD VESSEL DEPTH ESTIMATION UNIT ~85

BLOOD VESSEL THICKNESS MEASUREMENT UNIT ~91

DISPLAY CONTROL UNIT ~86

VIDEO SIGNAL CREATION UNIT ~68

16A

MONITOR ~18

EP 3 446 617 A1

## INTERNATIONAL SEARCH REPORT

| International application No. |
| --- |
| PCT/JP2017/007665 |

| A. CLASSIFICATION OF SUBJECT MATTER |
| --- |
| *A61B1/00*(2006.01)i, *A61B1/045*(2006.01)i |

According to International Patent Classification (IPC) or to both national classification and IPC

| B. FIELDS SEARCHED |
| --- |

Minimum documentation searched (classification system followed by classification symbols)
A61B1/00, A61B1/045

Documentation searched other than minimum documentation to the extent that such documents are included in the fields searched

| Jitsuyo Shinan Koho | 1922–1996 | Jitsuyo Shinan Toroku Koho | 1996–2017 |
| Kokai Jitsuyo Shinan Koho | 1971–2017 | Toroku Jitsuyo Shinan Koho | 1994–2017 |

Electronic data base consulted during the international search (name of data base and, where practicable, search terms used)

| C. DOCUMENTS CONSIDERED TO BE RELEVANT |
| --- |

| Category* | Citation of document, with indication, where appropriate, of the relevant passages | Relevant to claim No. |
| --- | --- | --- |
| X<br>A | JP 2012-125461 A (Fujifilm Corp.),<br>05 July 2012 (05.07.2012),<br>paragraphs [0059] to [0063]<br>& US 2012/0154566 A1<br>paragraphs [0115] to [0120]<br>& EP 2466874 A2 | 1-3,17-18<br>4-16 |
| A | JP 2011-167349 A (Olympus Medical Systems Corp.),<br>01 September 2011 (01.09.2011),<br>paragraphs [0038] to [0051]<br>(Family: none) | 1-18 |

| ☒ Further documents are listed in the continuation of Box C. | ☐ See patent family annex. |
| --- | --- |

| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>15 May 2017 (15.05.17) | Date of mailing of the international search report<br>23 May 2017 (23.05.17) |
| --- | --- |
| Name and mailing address of the ISA/<br>Japan Patent Office<br>3-4-3,Kasumigaseki,Chiyoda-ku,<br>Tokyo 100-8915,Japan | Authorized officer<br><br>Telephone No. |

Form PCT/ISA/210 (second sheet) (January 2015)

## INTERNATIONAL SEARCH REPORT

International application No.

PCT/JP2017/007665

C (Continuation).    DOCUMENTS CONSIDERED TO BE RELEVANT

| Category* | Citation of document, with indication, where appropriate, of the relevant passages | Relevant to claim No. |
|---|---|---|
| A | JP 2014-23591 A  (Fujifilm Corp.), 06 February 2014 (06.02.2014), paragraph [0054]; fig. 11 & US 2014/0031628 A1 paragraph [0095]; fig. 11 & EP 2689712 A1 | 1-18 |
| A | JP 2010-51350 A  (Fujifilm Corp.), 11 March 2010 (11.03.2010), paragraph [0038] & US 2010/0054576 A1 paragraph [0060] | 1-18 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5393525 B

- JP 2011218135 A